(19) Europäisches Patentamt
European Patent Office
Office européen des brevets

(11) **EP 3 968 025 A1**

(12) **EUROPEAN PATENT APPLICATION**

(43) Date of publication:
**16.03.2022 Bulletin 2022/11**

(21) Application number: **20306031.4**

(22) Date of filing: **15.09.2020**

(51) International Patent Classification (IPC):
**G01N 33/574** [(2006.01)]

(52) Cooperative Patent Classification (CPC):
**G01N 33/57407**; G01N 2800/52

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Institut Gustave Roussy**
**94800 Villejuif (FR)**

(72) Inventor: **The designation of the inventor has not yet been filed**

(74) Representative: **Regimbeau**
**20, rue de Chazelles**
**75847 Paris Cedex 17 (FR)**

(54) **METHODS FOR THE DETERMINATION OF INVASIVE ABILITY OF BRAIN TUMOUR CELLS AND FOR THE DIAGNOSIS AND PROGNOSIS OF BRAIN TUMOUR**

(57) The present invention concerns a novel *in vitro* assay for determining the invasive ability of brain tumour cells, in particular brain tumour stem cells. This new assay, called 3D invasion assay, is based on the analysis and comparison of the area of neurospheres grown *in vitro* into a Matrigel extracellular-like matrix. The invention also concerns the use of said assay for diagnosing metastatic brain tumour in subjects, for determining its prognosis, as well as for the monitoring, for the stratification, for identifying subjects at risk of metastasis, and/or predicting the metastasis-associated risk in subjects diagnosed with brain tumour.

EP 3 968 025 A1

Description

## SUMMARY OF THE INVENTION

The present Inventors have developed an original and powerful assay allowing to determine the invasive ability of brain tumour cells, in particular glioma. This new assay, called 3D invasion assay, is based on the analysis and comparison of the area of neurospheres grown *in vitro* into a Matrigel matrix. It can be used to rapidly and reliably predict the inter-individual risk of metastasis development in patients suffering from brain tumour, such as glioma. When using this assay, a remarkable correlation was observed between *in vitro* data and clinical profiles. Importantly, this assay, reproducing the physiological interaction with extracellular matrix, is more sensitive and more specific in predicting metastasis risk than the tests classically used for determining cell migration or invasion abilities (Chemotaxis assay, also called Boyden-like assay, and scratch-wound assay).

## BACKGROUND ART

Gliomas are a type of brain tumour that originate from glial progenitor cells of the brain or the spine. Gliomas comprise about 50 percent of all brain tumours and central nervous system tumours, and represent the most frequent malignant brain tumours in adults but also in children.

Diffuse midline gliomas (DMGs) account for about 20% of childhood brain cancers and are a leading cause of cancer mortality in children, adolescents and young adults, with a median survival of 9-12 months in patients with these tumours (1-3). These tumours are highly invasive and approximately 30-40% of patients develop intracranial metastases during disease progression (4,5).

The deep localization and infiltrating nature of these tumours render them inoperable, and the numerous clinical trials in recent years have not made it possible to discover new effective treatments for the management of this pathology; radiotherapy, which remains the only recognized treatment, is only transiently effective (6). During their progression, a considerable proportion of patients develop metastatic foci in different regions of their brain, which will only be identified at relapse or autopsy (4). The formation of metastases represents a very poor prognostic factor in these patients. The very early identification of subjects with an increased risk of developing metastases could influence the adaptation of the treatment regimen with the use of whole-brain or cranio-spinal radiotherapy to reduce the risk of distant metastatic foci. In this context, no test can, at present, predict early and reliably the individual risk for each patient to develop metastases during the course of the disease (7).

Tests have been developed in order to analyse the mobility of cells *in vitro,* such as chemotaxis assays, also called Boyden-like assay, and scratch-wound assays (8,9). However, these tests are not completely representative of the physiological context in which tumours such as gliomas develop and progress. Indeed, the correlation between clinical data and *in vitro* data obtained with these tests is low, and these tests do not allow to predict the invasive ability of the tumour with high sensitivity, specificity and reliability.

Therefore, there is a pressing need to identify more reliably and at earlier stages, cancerous patient that are at risk of progressing to metastasis (7). This will lead to an earlier, quicker and more effective management of these patients, thereby limiting metastasis progression, and ultimately allowing an amelioration of the overall survival and a decrease in the mortality rate.

The present invention fulfils this need. Indeed, the present Inventors have set-up an original method based on a 3D invasion assay to reliably quantify the invasiveness of biopsy-derived tumour stem cells and predict the risk of metastatic relapse. Using this methodology, the inventors showed a strong correlation between the risk of metastasis at relapse in patients and the invasiveness of primary models derived from biopsies at diagnosis. Indeed, the Inventors demonstrated that the models derived from patients who developed metastases during their progression showed a very high invasiveness *in vitro,* whereas invasiveness from models derived from patients without metastatic progression was significantly lower.

The present invention thus provides novel, sensitive and efficient tools for prognosing brain tumours (such as glioma), for monitoring and/or for the stratification of subjects suffering from a brain tumour (such as glioma) as well for identifying subjects at risk of metastasis, and/or for predicting the metastasis-associated risk, in subjects diagnosed with a brain tumour (such as glioma).

## DETAILED DESCRIPTION OF THE INVENTION

### Definitions

Unless specifically defined, all technical and scientific terms used herein have the same meaning as commonly understood by a skill artisan in chemistry, biochemistry, cellular biology, molecular biology, and medical sciences.

As used herein, a **"brain tumour"** is a tumour occurring in the brain. A brain tumour occurs when abnormal cells form within the brain. There are two main types of tumours: cancerous (malignant) tumours and benign (non-cancerous) tumours. Cancerous tumours can be divided into primary tumours, which start within the brain, and secondary tumours, which most commonly have spread from tumours located outside the brain, known as brain metastasis tumours. Examples of the most common primary brain tumours include gliomas (about 50% of the brain tumours), meningiomas (about 20%), pituitary adenomas (about 15%), nerve sheath tumours (about 8% of the brain tumours), etc.

As used herein, a **"glioma"** is a general name for any tumour that arises from the supportive tissue called glia, which help keep the neurons in place and functioning well. A glioma thus starts in the glial cells of the brain or the spine. Gliomas comprise about 50% of all brain tumours and central nervous system tumours, and the most frequent among all malignant brain tumours. Gliomas can be classified by cell type, by grade, and by location.

In the WHO (World Health Organisation) classification, gliomas are named according to the specific type of cell with which they share histological features, but not necessarily from which they originate (2,3). The main types of gliomas are:

- Ependymomas: ependymal cells;
- Astrocytomas: astrocytes (glioblastoma multiforme is a malignant astrocytoma and the most common primary brain tumour among adults);
- Oligodendrogliomas: oligodendrocytes;
- Diffuse midline gliomas (DMG): introduced as a completely new entity in the revised 2016 WHO classification of tumours of the central nervous system (CNS). DMG likely develop from oligodendroglial progenitor cells and share the same type of driver molecular alterations disrupting the function of the chromatin organizing complex PRC2 (1);
- Mixed gliomas, such as oligoastrocytomas and gangliogliomas, contain different types of glial cells with some uncertainties about the cell of origin.

Gliomas can be further categorized according to their grade, which is determined by pathologic evaluation of the tumour. The neuropathological evaluation and diagnostics of brain tumour specimens can be performed according to WHO Classification of Tumours of the Central Nervous System:

- Biologically benign gliomas [WHO grade I] are comparatively low risk and can be removed surgically depending on their location;
- Low-grade gliomas [WHO grade II] are well-differentiated (not anaplastic); these tend to exhibit benign tendencies and portend a better prognosis for the patient. However, they have a uniform rate of recurrence and increase in grade over time so should be classified as malignant;
- High-grade [WHO grades III-IV] gliomas are undifferentiated or anaplastic; these are malignant and carry a worse prognosis. DMG are assigned to WHO grade IV.

As used herein, a **"glioblastoma"** or "glioblastoma multiforme" or **"GBM"** is the most aggressive type of brain cancer that begins within the brain. Glioblastoma is a type of astrocytoma (it is Grade IV astrocytoma according to WHO classification). Glioblastomas occur in both adults and children. Glioblastomas are characterized by the presence of small areas of necrotizing tissue that are surrounded by anaplastic cells. This characteristic, as well as the presence of hyperplastic blood vessels, differentiates the tumour from grade III astrocytomas, which do not have these features. GBMs usually form in the cerebral white matter, grow quickly, and can become very large before producing symptoms. Fewer than 10% form more slowly following degeneration of low-grade astrocytoma or anaplastic astrocytoma. These are called secondary GBMs and are more common in younger patients.

As used herein, a **"diffuse midline glioma"** or **"DMG"** or **"midline infiltrating glioma"** or **"MIG"** is a primary central nervous system (CNS) tumour (2). Diffuse midline glioma is a rare subtype of glial tumours (glioma). They grow in the areas of the brain that are found in the midline. They most commonly grow in the pons (middle) in region of the brain stem, the thalamus and the spinal cord. Diffuse midline gliomas are more common in children, but they can occur in adults. They are usually fast growing, likely to spread, sometimes referred to as malignant or cancerous (5). They are in most cases difficult to remove surgically because they are diffuse, which means they don't have well-defined borders and tumour cells are interspersed within the healthy tissue of the brain. Diffuse midline gliomas are particularly resistant to radiotherapy, whose effectiveness is only transient, and are chemo-resistant (6).

A **"diffuse intrinsic pontine glioma (DIPG)"** is a particular subtype of DMG originating in the pons (middle) of the brain stem. DIPG is a brainstem glioma. The majority of brain stem tumours occur in the pons and are diffusely infiltrating (they grow amidst the nerves), and are therefore difficult to be surgically removed (4,5,7).

As used herein, **"midline"** or **"brain midline"** is the natural centre line of the brain between its two halves. As used herein, **"brainstem"** or **"brain stem"** is the posterior part of the brain, continuous with the spinal cord, connecting the cerebrum with the spinal cord. In the human brain the brainstem is composed of the midbrain, the pons, and the medulla oblongata. The brainstem is a very small component of the brain, making up only around 2.6 percent of its total weight. It has the critical role of regulating cardiac and respiratory function, helping to control heart rate and breathing rate. It also provides the main motor and sensory nerve supply to the face and neck via the cranial nerves. Other roles include the regulation of the central nervous system and the body's sleep cycle. It is also of prime importance in the conveyance of motor and sensory pathways from the rest of the brain to the body, and from the body back to the brain.

As used herein, a **"brain tumour cell"** is any type of cell that can be found in a brain tumour. The brain tumour cell is preferably a cancerous cell (in particular a glioma cell, more particularly a glioblastoma cell). As used herein a **"brain tumour stem cell"** is a sub-population of tumour cells with the key abilities of their normal counterpart, i.e. non-tumoral adult stem and progenitor cells. Indeed, tumour stem cells are able to self-renew their own pool and sustain tumour growth and phenotypic heterogeneity. The brain tumour stem cell is preferably a GSC.

As used herein, a **"glioma cell"** is any type of cell that can be found in a glioma tumour. The glioma cell is preferably a cancerous cell. Advantageously, glioma cells comprise glioma stem cells (GSCs). As used herein a **"glioma stem cell"** or **"GSC"** is a subpopulation of glioma cells. They are glioma tumorigenic cancer stem cells (CSCs) that contribute to tumour initiation and therapeutic resistance of gliomas to conventional therapies as well as recurrent disease. As normal stem and progenitor cells participate in tissue development and repair, these developmental programs re-emerge in CSCs to support the development and progressive growth of tumours. GSCs are capable of self-renewal and differentiation; glioblastoma-derived GSCs are capable of de novo tumour formation when implanted in xenograft models (10). Further, GSCs possess unique surface markers (e.g. CD133 (or prominin-1), L1 cell adhesion molecule (or L1CAM, or CD171) CD15, Nestin or A2B5 which allow to identify these cells that are otherwise characterized by their ability to grow as sphere in serum-free neurobasal medium and to form tumours in animals with as little as 100 cells in athymic (immunodeficient) rodents. GSCs modulate characteristic signalling pathways to promote tumorigenesis and play key roles in glioma vascular formation.

As used herein, **"metastasis"** is a tumour and/or cancer spread from an initial or primary site to a different or secondary site within the host's body. The newly pathological sites are called **"metastases"** or **"secondary tumours"** or **"metastatic tumours"** (those terms are herein equivalent).

As used herein, a **"metastatic glioma"** or a **"metastatic form of glioma"** is a glioma which has undergone or is undergoing metastasis. A **"metastatic glioblastoma"** or a **"metastatic form of glioblastoma"** is a glioblastoma which has undergone or is undergoing metastasis. A **"metastatic DMG"** or a **"metastatic form** of **DMG"** is a DMG which has undergone or is undergoing metastasis.

As used herein, a **"subject"** or an **"individual"** is an animal, preferably a mammal, including, but not limited to, human, dog, cat, cattle, goat, pig, swine, sheep and monkey. More preferably, the subject is a human subject. A human subject can be known as a patient. The human subject may be a child or an adult. As used herein, **"subject in need"** refers to an animal, preferably a mammal, more preferably a human, that may suffer from, or susceptible to suffer from a brain tumour, in particular a glioma, more particularly a glioblastoma or a DMG; or that is suspected of suffering from a brain tumour, in particular glioma, more particularly a glioblastoma or a DMG; or that has been diagnosed with a brain tumour, in particular glioma, more particularly a glioblastoma or a DMG.

As used herein, a **"brain tumour suffering subject"** refers to an animal, preferably a mammal, more preferably a human, that suffers from a brain tumour and/or has been diagnosed with a brain tumour. As used herein, a **"glioma suffering subject"** refers to an animal, preferably a mammal, more preferably a human, that suffers from glioma and/or has been diagnosed with glioma.

As used herein, a **"glioblastoma suffering subject"** refers to an animal, preferably a mammal, more preferably a human, that suffers from glioblastoma and/or has been diagnosed with glioblastoma. As used herein, a **"DMG suffering subject"** refers to an animal, preferably a mammal, more preferably a human, that suffers from DMG and/or has been diagnosed with DMG.

"Brain tumour patients" are patients (preferably human subjects) diagnosed with a brain tumour. **"Glioma patients"** are patients (preferably human subjects) diagnosed with glioma (e.g. "glioblastoma patients", "DMG patients", etc.). Diagnosis of these types of cancers can be done by using known imaging techniques enabling to detect the tumour (such as Magnetic resonance imaging (MRI)).

A **"control subject"** or a **"reference subject"** is a subject that may either be a healthy subject or a subject suffering from a disease (preferably a brain tumour, more preferably a glioma, in particular a glioblastoma or a DMG, more particularly a DMG) at a specific stage (e.g. a benign brain tumour [WHO grade I], or a low-grade brain tumour [WHO grade II], or a high-grade brain tumour [WHO grades III-IV], and/or a metastatic form of brain tumour; such as a benign glioma [WHO grade I], or a low-grade glioma [WHO grade II], or a high-grade glioma [WHO grades III-IV], and/or a metastatic form of glioma; such as a benign glioblastoma [WHO grade I], or a low-grade glioblastoma [WHO grade II], or a high-grade glioblastoma [WHO grades III-IV], and/or a metastatic form of glioblastoma; such as a benign DMG, or a low-grade DMG, or a high-grade DMG, and/or a metastatic form of DMG).

As used herein, the terms **"biological sample"** or **"sample"** refer to an entire organ or tissue of a subject or a portion thereof, or a fluid or fraction thereof, or cells or cell components thereof, or a fraction or portion of tissue, organ, fluid or cell. "Biological sample" further refers to a homogenate, lysate or extract prepared from an entire organ or tissue or fluid of a subject or portions thereof, or cells or cell components thereof, or a fraction or portion of tissue, organ, fluid or cell. In particular, a **"biological sample"** or **"sample"** is any tissue (preferably portions or fractions thereof) which may contain brain tumour cells (in particular brain tumour stem cells), preferably glioma cells (in particular GSCs), including, but not limited to, a central nervous system (CNS) sample, such as a brain sample or a spinal cord sample. More particularly, a "biological sample" or "sample" is a tumour sample, preferably a central nervous system (CNS) tumour sample, such as a brain tumour sample or a spinal cord tumour sample, and more particularly a brain tumour sample.

The biological sample can be obtained by any technique known in the art. Such techniques include, for example, surgery (such as stereotactic surgery), puncture, explant, excision, biopsy. **"Excision"** herein means a surgical procedure consisting in cutting (excising) a more or less wide or deep part of the tissue, preferably an abnormality or growth of the tissue. An excision may be performed to remove and/or analyse a cancerous or suspicious tumour. **"Biopsy"** herein refers to a sample of cells or tissue taken for analysis. Several types of biopsy procedures are known and practiced in the field. The most common types include (1) incisional biopsy, in which only a sample of the tissue is taken; (2) excisional biopsy (or surgical biopsy), which consists of the total removal of a tumour mass, thus performing a therapeutic and diagnostic procedure; and (3) needle biopsy, in which a tissue sample is taken with a needle, which can be large or thin. Other types of biopsy exist, such as smears or curettage, and can also be used to obtain the sample. As a consequence, the sample can be, for example, an explant, or an excision, or a biopsy, etc. The sample is preferably obtained by a minimally invasive procedure, such as stereotactic surgery.

**"Stereotactic surgery"** is a minimally invasive form of surgical intervention which makes use of a three-dimensional coordinate system to locate small targets inside the body and to perform on them actions such as explant, excision, ablation, biopsy, etc.

As used herein, **"diagnosis"** or **"identifying a subject having"**, or **"diagnosing"** refers to a process of determining if a subject is afflicted with a disease, condition or ailment (in particular a cancer and/or a tumour, e.g. a brain tumour, preferably glioma, in particular glioblastoma or DMG, more particularly DMG). "Diagnosis" means the identification/determination of a disease (or condition, or ailment), or the absence of a disease (or condition, or ailment), in a subject. Diagnosis includes, for example, the investigation of the causes (etiology) and effects (symptoms) of the disease, in particular on the basis of observations and/or measurements, carried out using various tools.

More specifically, **"diagnosing a brain tumour"** refers to the process of determining whether a subject is suffering from a brain tumour or not. **"Diagnosing glioma"** refers to the process of determining whether a subject is suffering from a glioma or not (e.g. a glioblastoma or DMG). In particular, **"diagnosing DMG"** refers to the process of determining whether a subject is suffering from DMG.

**"Prognosis"** herein means the prediction/determination/assessment of the risk of disease (in particular a cancer and/or a tumour) progression (or evolution, or development) in an individual. Prognosis includes the assessment of the future development of the subject's condition and the possible chances of cure. The prognosis can be determined on the basis of observations and/or measurements, carried out using various tools.

As used herein, **"stratification"** refers to the separation/classification of subjects into subgroups by disease (in particular a cancer and/or a tumour) severity. The different subgroups include the subgroup of healthy subjects as well as different subgroups of subjects with a disease, classified according to the stage of disease progression/advancement/severity (in the present case, WHO grade I, II, III or IV brain cancer such as glioma, in particular glioblastoma or DMG, more particularly DMG ; and/or metastatic or non-metastatic brain cancer, such as glioma, in particular glioblastoma or DMG, more particularly DMG).

**"Monitoring"** or **"Follow-up"** herein refers to the identification/assessment of the progression (or evolution, or development) of a disease (in particular a cancer and/or a tumour) in a subject. Monitoring may be carried out on the basis of observations and/or measurements, using different tools, at different time intervals. Intervals may be regular or irregular. Their frequency depends on the disease but also on the stage of disease progression. It can range from a few days (e.g. in case of severe/advanced/severe disease and/or rapidly progressing disease and/or exacerbation phase) to a few years (e.g. in case of early, mild or moderate disease and/or slowly progressing disease).

As used herein, **"determining the outcome"** or **"outcome determination"**, herein means the prediction/determination/assessment of the most probable evolution (or progression or development) of a disease (in particular a cancer and/or a tumour) in a subject. **"Determining the outcome"** of the disease thus includes at least assessing the next stages that are most likely to be undergone by the subject, in terms of probability. More specifically, **"determining the outcome of a brain tumour"** includes, but is not limited to, the assessment of the probabilities (or chances), for a subject (suffering from a brain tumour or not), of switching to a metastatic form; and/or the assessment of the probabilities (or chances), for a subject (suffering from brain tumour or not), of having or progressing towards a brain tumour and/or a metastatic brain tumour. In particular, **"determining the outcome of glioma (e.g. glioblastoma or DMG)"** includes, but is not limited to, the assessment of the probabilities (or chances), for a subject (suffering from glioma (e.g. glioblastoma) or not), of switching to a metastatic form; and/or the assessment of the probabilities (or chances), for a subject (suffering from glioma (e.g. glioblastoma) or not), of having or progressing towards a glioma (e.g. glioblastoma or DMG) and/or a metastatic glioma (e.g. glioblastoma or DMG). More particularly, **"determining the outcome of DMG"** includes, but is not limited to, the assessment of the probabilities (or chances), for a subject (suffering from DMG or not), of switching to a metastatic DMG form; and/or the assessment of the probabilities (or chances), for a subject (suffering from DMG or not), of having or progressing towards a DMG, and/or a metastatic form of DMG.

As used herein, **"predicting the metastasis-associated risk"** or **"assessing the risk of switching to a metastatic form"** means the prediction/determination/assessment of the probabilities (or the chances) of exacerbation (or aggravation, or intensification) of a cancer and/or a tumour, in particular the prediction/determination/assessment of the probabilities (or the chances) to switch from a non-metastatic to a metastatic form. More specifically, **"predicting the metastasis-associated risk of a brain tumour"** or **"assessing the risk of switching to a metastatic brain tumour form"** means the prediction/determination/assessment of the probabilities (or the chances) of exacerbation of brain tumour from a non-metastatic to a metastatic form. In particular, **"predicting the metastasis-associated risk of a glioma (e.g. glioblastoma or DMG)"** or **"assessing the risk of switching to a metastatic glioma (e.g. glioblastoma or DMG) form"** means the prediction/determination/assessment of the probabilities (or the chances) of exacerbation of glioma (e.g. glioblastoma or DMG) from a non-metastatic to a metastatic form. More particularly, **"predicting the metastasis-associated risk of a DMG"** or **"assessing the risk of switching to a metastatic DMG form"** means the prediction/determination/assessment of the probabilities (or the chances) of exacerbation of DMG from a non-metastatic to a metastatic form.

As used herein, the terms **"prevent"** or **"preventing"** or **"prevention"** or **"prevention of the onset of a disease"** means the reduction of the risk of appearing, of developing or of amplifying for a disease, for the causes of a disease, for the symptoms of a disease, for the effects (or consequences, preferably adverse, deleterious effects/consequences) of a disease, or any combination thereof ; and/or delaying the onset, development or amplification of a disease, the causes of a disease, the symptoms of a disease, the effects (or consequences, preferably adverse, deleterious effects/consequences) of a disease, or any combination thereof. In the present case, "preventing metastatic brain tumour" or "preventing metastatic glioma (e.g. glioblastoma or DMG)" comprises reducing the likelihood that the subject undergoes the switch into a metastatic form.

As used herein, the terms **"treat", "treating", "treatment"** and the like mean the reduction, inhibition, amelioration, stabilization and/or disappearance of a disease (or an ailment, or a condition), of the causes of a disease, of the symptoms (or signs) of a disease, of the effects (or consequences, preferably adverse, deleterious effects/consequences) of a disease (e.g. (in particular a cancer and/or a tumour, such as a brain tumour, in particular glioma, more particularly glioblastoma and DMG, and/ or symptoms associated therewith), fighting the disease, or any combination thereof. It will be appreciated that, although not precluded, treating a disorder or condition does not require that the disorder, condition or symptoms associated therewith be completely eliminated. For examples, treating results in the reduction of at least one sign or symptom of the disease or condition. Treatment includes (but is not limited to) administration of a therapy, and may be performed either prophylactically, or subsequent or the initiation of a pathologic event. Treatment can require administration of a therapy more than once.

More specifically, **"treating a brain tumour"** (such as **"treating a glioma", "treating a glioblastoma",** and/or **"treating DMG")** refers to fighting at least one brain tumour (such as glioma, glioblastoma, and/or DMG, respectively) in a human or animal organism. Advantageously, the size of the brain tumour (e.g. a glioma tumour, in particular a glioblastoma tumour or a DMG tumour) in the organism will decrease, within a shorter period of time than expected without treatment. The terms **"treating a brain tumour"** (such as **"treating a glioma", "treating a glioblastoma",** and/or **"treating DMG")** also refer to reducing/inhibiting/ameliorating/stabilizing/making disappear, the symptoms/signs associated with a brain tumour, such as glioma, glioblastoma, and/or a DMG, respectively).

As used herein, a **"therapy"** or **"treatment"** includes, but is not limited to, molecule(s) and/or drugs (including any type of molecule or drug, such as chemical or biological compounds, antibody, antigen, gene therapy, cell therapy, immunotherapy, chemotherapy, any combination thereof, etc.), and/or other treatments (such as radiotherapy, radiation, immunotherapy, chemotherapy, surgery, endoscopy, interventional radiology, physical oncology, phototherapy, light therapy, ultrasound therapy, thermotherapy, cryotherapy, electrotherapy, electroconvulsive therapy, oxygen therapy, assisted ventilation, hydrotherapy massage, organ/tissue/fluid transplant, implant, and any combination thereof, etc.). A therapy can be administered through various administration modes. The skilled person knowns how to select the most appropriate administration mode(s) depending on the therapy, the disease and the subject to treat. For example, administration modes include, but are not limited to, as oral administration; administration by injection into a vein (intravenously, IV), into a muscle (intramuscularly, IM), into the space around the spinal cord (intrathecally), beneath the skin (subcutaneously, sc); sublingual administration; buccal administration; rectal administration; vaginal administration; ocular route; otic route; nasal administration; by inhalation; by nebulization; cutaneous administration, either topical or systemic; transdermal administration.

In the case of brain tumours, such as gliomas, in particular glioblastoma and DMG, more particularly diffuse midline gliomas, the first treatment is surgery, if possible. The goal of surgery is to obtain tissue to determine the tumour type and to remove as much tumour as possible without causing more symptoms for the person. Diffuse midline gliomas usually require further treatments. Other treatments may include radiation, chemotherapy, or clinical trials. Surgery is followed by radiation therapy in most cases. Glioma, in particular glioblastoma and DMG, may also be treated with radiation therapy alone. Chemotherapy is sometimes given with radiation therapy, or after radiation.

As used herein, **"selecting a therapy"** or **"selecting a treatment"** or **"selecting a drug"** refers to the process of selecting (choosing, or deciding for, or opting for) the most appropriate therapy for a subject, in view of the symptoms (or signs) detected (observed and/or measured) in the subject, and/or in view of the subject himself, and the general knowledge in the medical field (preferably the medical field closest to the disease). Selecting a therapy include selecting the most appropriate therapy and may include also selecting the most appropriate administration mode and/or the most appropriate posology.

As used herein, the terms **"assessing the efficacy of a therapy"** or **"assessment of treatment efficacy"** refers to the determination of the clinical status of a subject undergoing treatment (i.e. receiving a therapy). The treatment may be preventive, for example in the case of predisposition to a disease, or it may be curative, for example in the case of a diagnosed disease. For example, the effectiveness of the treatment can be assessed by determining the condition of the subject at different time intervals. In particular, the condition of the subject can be assessed before the first dose of treatment and then at regular (or irregular) intervals after the first dose (e.g. after each new dose of treatment). A comparison of the subject's condition at these different intervals can then be made to identify any changes. The patient's condition can be assessed on the basis of observations and/or measurements made with different tools.

As used herein, the expressions **"invasive ability of a cell"** and **"metastatic ability of a cell"** refer to the capacity of a cell to migrate/circulate/move from one site (called primary site) to another, distinct, site(s) (called secondary site(s)), within a whole organism or an entire organ or a tissue or portions thereof. The primary site can be the organ or tissue wherein the cell originally arises within the organism. The primary site can be the place/site/location/position/spot/section wherein the cell originally arises within the organ or the tissue. The secondary site(s) can be a portion of the organism or the organ or the tissue that is distinct form the primary site. Advantageously, the secondary site is a place in the organism (such as an organ or a tissue) or in the organ or in the tissue wherein the cell should not be in normal/healthy/physiological conditions for the organism. The terms "invasive ability of brain tumour cells" and "invasive ability of glioma cells" means the invasive ability of cells of a brain tumour or of a glioma, respectively.

A **"primary cell model"** or **"primary culture"** or **"primary cell culture"** refers to is a cell culture system that is formed by culturing cells directly obtained from a biological sample (such as an organ or tissue or portions thereof, e.g. a biopsy). A **"brain tumour primary cell culture"** or a **"brain tumour primary culture"** or a **"glioma primary cell culture"** or a **"glioma primary culture"** is a cell culture that is formed by culture cells directly obtained from a tumour sample from a subject. The brain tumour primary cell model (such as a glioma primary culture) is preferably obtained from a sample of a central nervous system (CNS) tumour, such as a brain tumour sample or a spinal cord tumour sample, even more preferably from a glioma sample. Preferably, the brain tumour primary culture comprises, or consists essentially of, or consists of, brain tumour cells (such as glioma cells). More preferably, the brain tumour primary culture comprises, or consists essentially of, or consists of brain tumour stem cells (such as GSCs). Preferably, the glioma primary culture comprises, or consists essentially of, or consists of, glioma cells (such as glioblastoma cells or DMG cells, in particular DMG cells). More preferably, the glioma primary culture comprises, or consists essentially of, or consists of, GSCs.

As used herein, the term **"neurosphere"** or **"cell neurosphere"** means a culture system composed of free-floating clusters of cells comprising, or consisting essentially of, or consisting of, neural stem cells (11). As used herein, "neural stem cells" refers to NESTIN-expressing cells. As used herein, "neural stem cells" refers to sex determining region Y-box 2 (SOX2)-expressing cells. The neural stem cells are preferably brain tumour stem cells, more preferably GSCs, even more preferably GSCs obtained from a glioma (such as a glioblastoma or a DMG, in particular DMG). The neurosphere may also comprise other glioma cells. The term **"embedded neurosphere"** refers to a neurosphere which is embedded (thus not free floating anymore) in an appropriate medium. The appropriate medium is preferably a protein-based extracellular-like matrix.

The terms **"protein-based extracellular-like matrix"** refers to a matrix comprising, or consisting essentially of, or consisting of proteins. A matrix is an arrangement of molecules forming a network/polymer. The network can be more or less regular, more or less tight, more or less organised, more or less solid. This network allows to mimic *in vitro* the physiological complexity and stiffness of the extracellular matrix of tissues. The protein-based extracellular-like matrix is preferably gelatinous. Advantageously, the protein-based extracellular-like matrix is a matrix wherein the molecules forming the network/polymer (and/or organising the network/polymer) comprise, or consist essentially of, or consist of, proteins. In some embodiment, the protein-based extracellular-like matrix also comprises additional molecules. Advantageously, the protein-based extracellular-like matrix is initially in the form a solution (wherein the network/polymer/matrix has not form yet). In such case, the matrix can be formed for example by incubating at an appropriate temperature for an appropriate time, allowing polymerisation of the protein-based network. The protein-based extracellular-like matrix can thus be maintained in the form a solution by storing at an appropriate temperature (for example by freezing the matrix solution, at a temperature of -20°C or lower).

The protein-based extracellular-like matrix (and/or the protein-based extracellular-like matrix solution) preferably comprises, or consists essentially of, or consists of, basement membrane components. The **"basement membrane"** is a thin, fibrous, extracellular matrix that separates the lining of an internal or external body surface from underlying connective tissue in animals. This surface may be epithelium (skin, respiratory tract, gastrointestinal tract, etc.), mesothelium (pleural cavity, peritoneal cavity, pericardial cavity, etc.) and endothelium (blood vessels, lymph vessels, etc.). Basement membrane components include, but are not limited to, the following components: Laminin; Collagen, preferably Collagen type IV; Nidogen (also known as Entactin), preferably selected from the group consisting of Nidogen-1, Nidogen-2, and a combination thereof. The protein-based extracellular-like matrix may also comprise other components such as growth factor(s) and/or Heparan sulfate proteoglycan (also known as Perlecan).

**"Matrigel"** is the trade name for a gelatinous protein mixture secreted by Engelbreth-Holm-Swarm (EHS) mouse sarcoma cells. It is for example produced by Corning Life Sciences. Matrigel resembles the complex extracellular environment found in many tissues and is used by cell biologists as a substrate (basement membrane matrix) for culturing cells.

As used herein, **"Laminin"** refers to a high-molecular weight (~400 to ~900 kDa) protein of the extracellular matrix. Laminins are a major component of the basal lamina (one of the layers of the basement membrane), a protein network foundation for most cells and organs. The laminins are an important and biologically active part of the basal lamina, influencing cell differentiation, migration, and adhesion. Laminins are heterotrimeric proteins that contain an $\alpha$-chain, a $\beta$-chain, and a $\gamma$-chain, found in five, four, and three genetic variants, respectively. The laminin molecules are named according to their chain composition.

As used herein, **"Collagen"** refers to the main structural protein in the extracellular matrix in the various connective tissues in the body. As the main component of connective tissue, it is the most abundant protein in mammals, making up from 25% to 35% of the whole-body protein content. Collagen consists of amino acids bound together to form a triple helix of elongated fibril known as a collagen helix. It is mostly found in fibrous tissues such as tendons, ligaments, and skin. In human, Collagen has the NCBI reference sequence BAA04809.1 In mouse (*Mus musculus*), Collagen has the NCBI reference protein sequence NP_031764.2 or CAA47387.1 or NP_082046.2.

At least 30 types of collagen have been identified, described, and divided into several groups according to the structure they form. All of the types contain at least one triple helix. Collagen thus comprise Collage type I to type XXX. The Collagen is preferably Collagen type IV. **"Collagen type IV"** or **"Collagen IV"** or **"type-IV collagen"** or **"ColIV"** or **"Col4"** herein refers to a type of collagen found primarily in the basal lamina. The collagen IV C4 domain at the C-terminus is not removed in post-translational processing, and the fibres link head-to-head, rather than in parallel. Also, collagen IV lacks the regular glycine in every third residue necessary for the tight, collagen helix. This makes the overall arrangement sloppier with kinks. These two features cause the collagen to form in a sheet, the form of the basal lamina. Human Collagen IV is encoded by the following genes: COL4A1, COL4A2, COL4A3, COL4A4, COL4A5, COL4A6. In mouse (Mus musculus), Collagen IV protein has the NCBI reference sequence AAA50292.1 or AAA50293.1 or AAI38041.1 or BAB13674.1 or AAD50449.1.

As used herein, **"Nidogen"** or **"Entactin"** is a sulfated monomeric glycoprotein located in the basal lamina. Two nidogens have been identified in mammals: nidogen-1 (NID1) and nidogen-2 (NID2). Nidogen have been shown to play a crucial role during organogenesis in late embryonic development, particularly in cardiac and lung development. From an evolutionary perspective, nidogens are highly conserved across vertebrates and invertebrates, retaining their ability to bind laminin. Nidogen is preferably selected from the group consisting of Nidogen-1, Nidogen-2, and a combination thereof. In human, Nidogen-1 has the NCBI reference protein sequence EAW70041.1 or EAW70040.1 or EAW70039.1 and Nidogen-2 has the NCBI reference protein sequence EAW65658.1. In mouse (*Mus musculus*), Nidogen-1 has the NCBI reference protein sequence AAI31670.1 and Nidogen-2 has the NCBI reference protein sequence AAH57016.1 or AAH54746.1.

As used herein, **"Heparan sulfate proteoglycan"** or **"Perlecan"** or **"PLC"** or **"basement membrane-specific heparan sulfate proteoglycan core protein (HSPG)"** is a large multidomain (five domains, labelled I-V) proteoglycan that binds to and cross-links many extracellular matrix (ECM) components and cell-surface molecules. Perlecan comprises a core protein of molecular weight 470 kDa to which three long chains (each approximately 70-100 kDa) of glycosaminoglycans (often heparan sulfate, HS, but can be chondroitin sulfate, CS) are attached. The core protein consists of five distinct structural domains. Perlecan is synthesized by both vascular endothelial and smooth muscle cells and deposited in the extracellular matrix. Perlecan is a key component of the vascular extracellular matrix, where it interacts with a variety of other matrix components and helps to maintain the endothelial barrier function. Perlecan is a potent inhibitor of smooth muscle cell proliferation and is thus thought to help maintain vascular homeostasis. Perlecan can also promote growth factor (e.g., FGF2) activity and thus stimulate endothelial growth and re-generation. Perlecan is highly conserved across species. In human, Perlecan has the NCBI reference protein sequence AAA52700.1. In mouse (Mus musculus), Perlecan has the NCBI reference protein sequence NP_032331.2 or XP_006538638.1 or XP_006538636.1.

As used herein, a **"growth factor"** is a naturally occurring substance capable of stimulating cellular growth, proliferation, healing, and/or cellular differentiation. Usually it is a protein or a steroid hormone. Growth factors are important for regulating a variety of cellular processes. Growth factors typically act as signalling molecules between cells. Examples are cytokines and hormones that bind to specific receptors on the surface of their target cells. The growth factor is preferably selected from the group consisting of: Transforming Growth Factor Beta (TGF-beta), Epidermal Growth Factor (EGF), Insulin-like Growth Factor 1 (IGF-1), Basic Fibroblast Growth Factor (bFGF), Tissue Plasminogen Activator, Platelet-Derived Growth Factor (PDGF), and any combination thereof.

As used herein, **"Transforming Growth Factor Beta"** or **"TGF-beta"** or **"TGF-$\beta$"** is a multifunctional cytokine belonging to the transforming growth factor superfamily that includes three different mammalian isoforms (TGF-$\beta$ 1 to 3, HGNC symbols TGFB1, TGFB2, TGFB3) and many other signalling proteins. TGF-$\beta$ proteins are produced by all white blood cell lineages. Activated TGF-$\beta$ complexes with other factors to form a serine/threonine kinase complex that binds to TGF-$\beta$ receptors. TGF-$\beta$ receptors are composed of both type 1 and type 2 receptor subunits. After the binding of TGF-$\beta$, the type 2 receptor kinase phosphorylates and activates the type 1 receptor kinase that activates a signalling cascade. This leads to the activation of different downstream substrates and regulatory proteins, inducing transcription of different target genes that function in differentiation, chemotaxis, proliferation, and activation of many immune cells. Advantageously, Transforming Growth Factor Beta is selected from the group consisting of TGF-$\beta$1, TGF-$\beta$ 2, TGF-$\beta$ 3, and any combination thereof. In human, TGF-beta has the NCBI reference protein sequence AAA36738.1. In mouse (Mus musculus), TGF-beta has the NCBI reference protein sequence AAA37674.1.

As used herein, **"Epidermal Growth Factor"** or **"EGF"** is a protein that stimulates cell growth and differentiation by binding to its receptor, EGFR. Human EGF is 6-kDa and has 53 amino acid residues and three intramolecular disulfide bonds. In human, EGF has the EGF has the NCBI reference protein sequence AAH92277.1 or AAH60741.1.

As used herein, **"Insulin-like Growth Factor 1"** or **"IGF-1"** or **"somatomedin C"** is a hormone similar in molecular structure to insulin which plays an important role in growth, and has anabolic effects in adults. IGF-1 consists of 70 amino acids in a single chain with three intramolecular disulfide bridges. IGF-1 has a molecular weight of 7,649 Daltons. IGF-1 is produced primarily by the liver. Production is stimulated by growth hormone (GH). Most of IGF-1 is bound to one of 6 binding proteins (IGF-BP). IGFBP-1 is regulated by insulin. In human, IGF-1 has the NCBI reference protein sequence NP_001104753.1 or NP_000603.1 or NP_001278790.1 or NP_000609.1 or NP_001104755.1. In mouse (Mus musculus), IGF-1 has the NCBI reference protein sequence AAH12409.1.

As used herein, **"Basic Fibroblast Growth Factor"** or **"bFGF"** or **"FGF2"** or **"FGF-$\beta$"** is a growth factor and signalling protein. It is synthesized primarily as a 155 amino acid polypeptide, resulting in an 18 kDa protein. However, there are four alternate start codons which provide N-terminal extensions of 41, 46, 55, or 133 amino acids, resulting in proteins of 22 kDa (196 aa total), 22.5 kDa (201 aa total), 24 kDa (210 aa total) and 34 kDa (288 aa total), respectively. Generally, the 155 aa/18 kDa low molecular weight (LMW) form is considered cytoplasmic and can be secreted from the cell, whereas the high molecular weight (HMW) forms are directed to the cell's nucleus. It is involved in a variety of biological processes, including embryonic development, cell growth, morphogenesis, tissue repair, tumour growth and invasion. In normal tissue, bFGF is present in basement membranes and in the subendothelial extracellular matrix of blood vessels. It stays membrane-bound as long as there is no signal peptide. In human, bFGF has the NCBI reference protein sequence NP_001348594.1 or NP_001997.5. In mouse (Mus musculus), bFGF has the NCBI reference protein sequence XP_006535462.1 or NP_032032.1.

As used herein, **"Platelet-Derived Growth Factor"** or **"PDGF"** is a growth factor stimulating cell division (mitogen) in different cell types, such as cells in connective tissue and developing nervous system. It is formed by dimerized subunits of two out of five different polypeptides (subunits), namely AA chain or PDGF-AA (211 aa total), BB chain or PDGF-BB (241 aa total), AB, CC, DD, stabilized by disulphide bonds. PDGF stimulates cell growth by binding to its surface receptors **(PDGFR)** There also exist two distinct subunits of the PDGF receptor that also occurs in the cell membrane as a homodimer ($\alpha\alpha$, BB) or a heterodimer ($\alpha\beta$). In human, PDGF has the NCBI reference protein sequence NP_002598.4 (subunit chain A); PDGF has the NCBI reference protein sequence NP_002599.1 (subunit chain B).

As used herein, **"Tissue Plasminogen Activator"** or **"tPA'** is a protein involved in the breakdown of blood clots. It is a serine protease (EC 3.4.21.68) found on endothelial cells, the cells that line the blood vessels. As an enzyme, it catalyses the conversion of plasminogen to plasmin, the major enzyme responsible for clot breakdown. Human tPA has a molecular weight of -70 kDa in the single-chain form. In human, tPA has the NCBI reference protein sequence AAO34406.1 or CAX11668.1 or CAA00642.1. In mouse (Mus musculus), tPA has the NCBI reference protein sequence AAA40470.1.

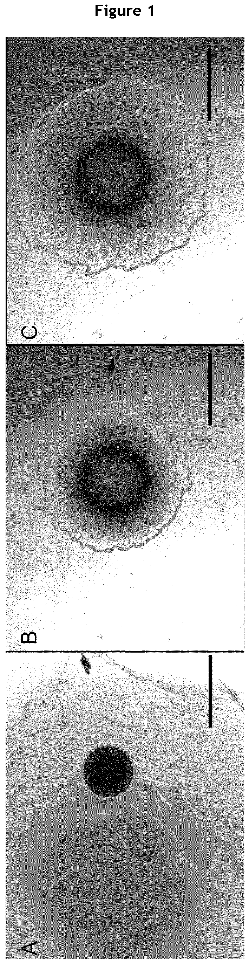

The terms **"measuring the area of a neurosphere"** or **"measuring the area of an embedded neurosphere"** herein means the act of evaluating the two-dimensional surface occupied by a neurosphere at any given time point post-embedding. This evaluation can be made at different timing using freely-available softwares (e.g. Image J, e.g. FiJi) on previously acquired two-dimensional photomicrographs of the embedded neurosphere(s) at perfect focus (examples of such acquired two-dimensional photomicrographs are given in Figure 1). Measuring the area of neurospheres during time has been used by researchers to evaluate the ability of invasion of glioma cells derived from different tumoral portions or tumour sub-clones (12,13)

As used herein, **"non-invasive analytical technique"** is a technique allowing to analyse/study a biological sample, such as a cell or a group of cells (in particular a neurosphere, more particularly an embedded neurosphere), while causing the less disturbance possible to the biological sample, preferably without disturbing the biological sample. Such techniques are well known in the art. The non-invasive analytical technique is preferably an imaging technique including, but not limited to microscopy (including confocal microscopy, fluorescence microscopy, electron microscopy, atomic force microscopy), methods based on the use of one or more excitation wavelengths and an appropriate optical method, as an electrochemical method (voltammetry and amperometry techniques), and radiofrequency methods, such as multipolar, confocal and non-confocal resonance spectroscopy, such as fluorescence, luminescence, chemiluminescence, absorbance, reflectance, transmittance, birefringence or refractive index (for example, by surface plasmon resonance, by ellipsometry, by resonant mirror method, etc.), radioisotopic or magnetic resonance imaging.

**Method of the invention, for determining the invasive ability of brain tumour cells (3D invasion assay)**

The present Inventors have developed an original and powerful assay, allowing to determine the invasive ability of brain tumour cells, in particular glioma cells, more particularly glioma stem cells (GSCs). The Inventors showed that this assay, also called 3D invasion assay, can be used to rapidly and reliably predict the risk of metastasis development in patients with a brain tumour such as glioma, with both high specificity and high sensitivity (higher than 80%). More precisely, comparative data showed that this 3D invasion assay is both more sensitive and more specific than the tests classically used for determining cell migration and invasive abilities (i.e. chemotaxis assay, also called Boyden-like assay and scratch-wound assay) in predicting metastasis risk associated with patients in a given cohort.

Accordingly, the present invention relates to a novel *in vitro* method for determining the invasive ability of brain tumour cells, in particular brain tumour stem cells, said method comprising the steps of:

a) obtaining a neurosphere from a brain tumour primary cell culture, wherein the brain tumour primary cell culture and/or the neurosphere preferably comprise(s) brain tumour stem cells;
b) embedding the neurosphere of step a) in a protein-based extracellular-like matrix, preferably using a solution of the protein-based extracellular-like matrix;
c) measuring the area of the embedded neurosphere of step b), preferably using a non-invasive analytical technique, such as an imaging technique;
d) incubating the embedded neurosphere of step c) at a temperature ranging from 35°C to 39°C (preferably at a temperature ranging from 36°C to 38°C, more preferably at a temperature around 37° C, even more preferably at 37°C), for 1h to 72h (preferably for 2h to 56h, preferably for 3h to 54h, preferably for 4h to 50h, preferably for 5h to 48h, preferably for 6h to 46h, preferably for 10h to 42h, more preferably for 40h;
e) measuring the area of the embedded neurosphere after step d), preferably using the same technique used in step c);
f) Determining the invasive ability of the brain tumour cells, in particular of the brain tumour stem cells, by comparing the area measured at step c) to the area measured at step e).

In a preferred embodiment, the brain tumour cells are glioma cells (in particular glioma stem cells (GSCs)), more preferably glioblastoma cells (in particular glioblastoma stem cells) more preferably DMG cells (in particular DMG stem cells).

Accordingly, the present invention relates to a novel *in vitro* method for determining the invasive ability of glioma cells, in particular glioma stem cells (GSCs), comprising the steps of:

a) obtaining a neurosphere from a glioma primary cell culture, wherein the glioma primary cell culture and/or the neurosphere preferably comprise(s) GSCs;
b) embedding the neurosphere of step a) in a protein-based extracellular-like matrix, preferably using a solution of the protein-based extracellular-like matrix;
c) measuring the area of the embedded neurosphere of step b), preferably using a non-invasive analytical technique, such as an imaging technique;
d) incubating the embedded neurosphere of step c) at a temperature ranging from 35°C to 39°C (preferably at a temperature ranging from 36°C to 38°C, more preferably at a temperature around 37°C, even more preferably at 37°C), for 1h to 72h (preferably for 2h to 56h, preferably for 3h to 54h, preferably for 4h to 50h, preferably for 5h to 48h, preferably for 6h to 46h, preferably for 10h to 42h, more preferably for 40h ;
e) measuring the area of the embedded neurosphere after step d), preferably using the same technique used in step c);
f) Determining the invasive ability of the glioma cells, in particular of the GSCs, by comparing the area measured at step c) to the area measured at step e).

When the *in vitro* method is for determining the invasive ability of glioblastoma cells (in particular glioblastoma stem cells), step a) comprises obtaining a neurosphere from a glioblastoma primary cell culture, wherein the glioblastoma primary cell culture and/or the neurosphere preferably comprise(s) glioblastoma stem cells.

When the *in vitro* method is for determining the invasive ability of DMG cells (in particular DMG stem cells), step a) comprises obtaining a neurosphere from a DMG primary cell culture, wherein the DMG primary cell culture and/or the neurosphere preferably comprise(s) glioblastoma stem cells.

Advantageously, the neurosphere of step a) is obtained by using an ultra-low attachment cell support (e.g. an ultra-low attachment well of a well plate, such as a U-bottom ultra-low attachment 96-well plate (for example commercialized by Corning).

Step a) of obtaining a neurosphere from a brain tumour primary cell culture can comprise the following successive phases:

1) Obtaining a cell suspension (preferably a single cell suspension) from the brain tumour primary cell culture, in an appropriate cell medium;

2) Placing a volume of the cell suspension of phase 1) in a U-shaped well of a ultra-low attachment plate, wherein the volume of the cell suspension preferably comprises between 15000-25000 cells, preferably between 16000-24000 cells, preferably between 17000-23000 cells, preferably between 18000-22000 cells, preferably between 19000-21000 cells, more preferably around 20000 cells;

3) Growing the volume of the cell suspension of phase 2) for 48-72 hours, at a temperature ranging from 35°C to 39°C (preferably at a temperature ranging from 36°C to 38°C, more preferably at a temperature around 37°C, even more preferably at 37° C), until the cells form spheres (preferably round-shaped spheres with smooth edges).

When step a) comprises obtaining a neurosphere from a glioma primary cell culture (preferably a glioblastoma primary cell culture, more preferably a DMG primary cell culture), the above phase 1) comprises obtaining a cell suspension (preferably a single cell suspension) from the glioma primary cell culture (preferably the glioblastoma primary cell culture, more preferably the DMG primary cell culture, respectively), in an appropriate cell medium.

Preferably, the appropriate cell medium used in phase 1) of step a) is a neural stem cell expansion medium. An appropriate neural stem cell expansion medium preferably comprises (or consists essentially of, or consists of) a serum-free neurobasal medium adapted for the culture of brain stem and progenitor cells from normal tissues or tumour samples. It preferably contains cytokines, such as human recombinant EGF (rhEGF) (for example at 20 ng/mL) and/or human recombinant BFGF (rhbFGF) (for example at 20 ng/mL). When culturing DMG-derived GSC (e.g. for obtaining a neurosphere from a DMG primary cell culture), appropriate neural stem cell expansion medium preferably comprises human recombinant PDGF-AA (10 ng/mL) and human recombinant PDGF-BB (10 ng/mL) (10). Conditions for culturing adherent neural stem cells derived from normal brain and gliomas have been originally described by (14,15). A particularly preferred cell medium consists of a serum-free neurobasal stem cell expansion medium (e.g. Neurocult NS-A (for example commercialized by STEMCELL Technologies)) advantageously supplemented with human-basic FGF (20 ng ml⁻¹, for example commercialized by STEMCELL Technologies), human EGF (20 ng ml⁻¹, for example commercialized by STEMCELL Technologies), PDGF-AA (10 ng ml⁻¹, for example commercialized by Miltenyi Biotec), PDGF-BB (10 ng ml⁻¹, for example commercialized by Miltenyi Biotec) and heparin (2 $\mu$g ml⁻¹, for example commercialized by STEMCELL Technologies) (10).

Advantageously, phase 3) of step a) comprises growing the volume of the cell suspension of phase 2) under an atmosphere comprising from 16% to 26% oxygen ($O_2$), more preferably from 18% to 24% $O_2$, more preferably from 20% to 22% $O_2$, more preferably approximately 21% $O_2$, even more preferably 21% $O_2$. The atmosphere advantageously further comprises approximately from 2% to 8% carbon dioxide ($CO_2$), more preferably from 4% to 6% $CO_2$, more preferably approximately 5% $CO_2$, even more preferably 5% $CO_2$. Advantageously, the atmosphere further comprises approximately from 80% to 100% humidity, more preferably from 85% to 95% humidity, more preferably approximately 90% humidity, even more preferably 90% humidity.

The protein-based extracellular-like matrix (and/or the protein-based extracellular-like matrix solution, i.e. the protein-based extracellular-like matrix solution before polymerisation) used in step b) of the method of the invention preferably comprises, or consists essentially of, or consists of, basement membrane components. The basement membrane components that can be advantageously used in the protein-based extracellular-like matrix used in the invention are selected from the group consisting of Laminin, Collagen (preferably Collagen type IV), Nidogen (also known as Entactin, preferably selected from the group consisting of Nidogen-1, Nidogen-2), and a combination thereof. The protein-based extracellular-like matrix may also comprise other components such as growth factor(s) and/or Heparan sulfate proteoglycan (also known as Perlecan).

Advantageously, the protein-based extracellular-like matrix (and/or the protein-based extracellular-like matrix solution) used in step b) of the method of the invention preferably comprises Laminin.

In a particularly advantageous embodiment, the protein-based extracellular-like matrix used in step b) of the method of the invention is a liquid solution that become jellified after polymerization. This solution preferably comprises at least 10mg/mL of the proteins of the basement membrane components mentioned above, before polymerization and before adding the neurosphere. The protein-based extracellular-like matrix used in step b) preferably comprises at least 5mg/mL of proteins (final concentration), preferably between 4 and 12mg/mL, preferably between 6 and 10mg/mL, more preferably around 8mg/mL, even more preferably 8mg/mL (final concentration). In particular, the protein-based extracellular-like matrix used in step b) preferably comprises at least 5mg/mL of the proteins of the basement membrane components mentioned above (final concentration), preferably between 4 and 12mg/mL, preferably between 6 and 10mg/mL, more preferably around 8mg/mL, even more preferably 8mg/mL of the proteins of the basement membrane components mentioned above (final concentration). Indeed, the Inventors have shown that the test is particularly accurate in predicting the metastatic progression when the solution of the protein-based extracellular-like matrix used in step b) comprises, approximately 8mg/mL of proteins (final concentration).

Advantageously, the protein-based extracellular-like matrix (and/or the protein-based extracellular-like matrix solution) used in the method of the invention comprises at least one basement membrane component, wherein the basement membrane component is preferably selected from the group consisting of:

- Laminin;
- Collagen, preferably Collagen type IV (or Collagen IV);
- Nidogen (also known as Entactin), preferably selected from the group consisting of Nidogen-1, Nidogen-2, a combination thereof; and
- Any combination thereof.

The protein-based extracellular-like matrix (and/or the protein-based extracellular-like matrix solution) can also optionally further comprise at least one of the following components:

- at least one growth factor, preferably selected from the group consisting of: Transforming Growth Factor Beta (TGF-beta), Epidermal Growth Factor (EGF), Insulin-like Growth Factor 1 (IGF-1), Platelet Derived Growth Factor (PDGF), Fibroblast Growth Factor (bFGF), Tissue Plasminogen Activator, and any combination thereof;
- Heparan sulfate proteoglycan (also known as Perlecan).

In an advantageous embodiment, the protein-based extracellular-like matrix (and/or the protein-based extracellular-like matrix solution) used in the method of the invention comprises from 50% to 70% of Laminin (mass percentage) (preferably from 55% to 65% of Laminin, preferably from 56% to 64% of Laminin, preferably from 57% to 63% of Laminin, preferably from 58% to 62% of Laminin, preferably from 59% to 61% of Laminin, even preferably around 60% of Laminin) ; and/or from 2% to 14% Nidogen (mass percentage) (preferably from 3% to 13% of Nidogen, preferably from 4% to 12% of Nidogen, preferably from 5% to 11% of Nidogen, preferably from 6% to 10% of Nidogen, preferably from 7% to 9% of Nidogen, even preferably around 8% of Nidogen).

Preferably, the protein-based extracellular-like matrix (and/or the protein-based extracellular-like matrix solution) used in the method of the invention comprises, or consists essentially of, or consists of, a basement component extract, more preferably a basement component extract obtained from an Engelbreth-Holm-Swarm (EHS) mouse sarcoma.

In a more preferred embodiment, the protein-based extracellular-like matrix (and/or the protein-based extracellular-like matrix solution) can comprise, or consist essentially of, or consist of, Matrigel®.

In a preferred embodiment, step b) of the method of the invention (step of embedding the neurosphere) comprises two steps b1) and b2), wherein step b1) comprises incorporating the neurosphere in a solution of the protein-based extracellular-like matrix ; and step b2) comprises incubating said solution of step b1) at a temperature ranging from 35°C to 39°C (preferably at a temperature ranging from 36°C to 38°C, more preferably at a temperature around 37° C, even more preferably at 37°C)until the protein-based extracellular-like matrix solution is polymerized to form the protein-based extracellular-like matrix (for example incubating said solution for from 5 minutes to 45 minutes, preferably from 10 minutes to 40 minutes, preferably from 15 minutes to 35 minutes, preferably from 20 minutes to 30 minutes, more preferably for 25 minutes).

Advantageously, in step b2), the solution of step b1) is incubated under an atmosphere comprising from 16% to 26% oxygen ($O_2$), more preferably from 18% to 24% $O_2$, more preferably from 20% to 22% $O_2$, more preferably approximately 21% $O_2$, even more preferably 21% $O_2$. The atmosphere advantageously further comprises approximately from 2% to 8% carbon dioxide ($CO_2$), more preferably from 4% to 6% $CO_2$, more preferably approximately 5% $CO_2$, even more preferably 5% $CO_2$. Advantageously, the atmosphere further comprises approximately from 80% to 100% humidity, more preferably from 85% to 95% humidity, more preferably approximately 90% humidity, even more preferably 90% humidity.

In one advantageous embodiment, the non-invasive analytical technique of step c) and/or step e) is an imaging technique, preferably a microscopy technique, more preferably a technique using an inverted microscope.

In an advantageous embodiment, steps c) and/or e) of measuring the area of the embedded neurosphere comprise(s) performing measurements of the area using any freely-available software (e.g. Image J, e.g. FiJi) on acquired two-dimensional photomicrographs at perfect focus of the embedded neurosphere.

In an advantageous embodiment, steps c) and/or e) of measuring the area of the embedded neurosphere comprise(s) performing at least two measurements of the area of the embedded neurosphere (technical replicates). This can be done using any freely-available software (e.g. Image J, e.g. FiJi) on acquired two-dimensional photomicrographs at perfect focus of the embedded neurosphere for each measurement, and calculating the average area and/or the median area.

Preferably, step a) comprises obtaining more than one neurospheres from a glioma primary cell culture (e.g. at least two). In such case, step b) comprises embedding each of the neurospheres of step a) in the protein-based extracellular-like matrix (preferably separately, as distinct embedded neurospheres). Advantageously, in this embodiment, steps c) and/or e) comprise(s) measuring the area of each of the embedded neurospheres, and calculating the average neurosphere area and/or the median neurosphere area. For example, the average neurosphere area of step c) is the sum of the area measured at step c) for each neurosphere, divided by the total number of neurospheres measured. For example, the average neurosphere area of step e) is the sum of the area measured at step e) for each neurosphere, divided by the total number of neurospheres measured. For example, the median neurosphere area of step c) is the median of the area for each neurosphere measured at step c). For example, the median neurosphere area of step e) is the median of the area for each neurosphere measured at step e).

It is possible to combine this embodiment with making at least two, three, four, five, six, seven, eight, nine or at least ten measurements (or even more) of the area of each of the embedded neurosphere at perfect focus for each measurement, and calculating the average area and/or the median area, at step c) and/or step e). In this case, for example, the average neurosphere area of step c) is the sum of the average area calculated at step c) for each neurosphere, divided by the total number of neurospheres measured. For example, the average neurosphere area of step e) is the sum of the average area calculated at step e) for each neurosphere, divided by the total number of neurospheres measured. For example, the median neurosphere area of step c) is the median of the median area calculated for each neurosphere measured at step c). For example, the median neurosphere area of step e) is the median of the median area calculated for each neurosphere measured at step e).

In step d), the embedded neurosphere of step c) are preferably incubated until the next measurement (i.e. until step e)). Accordingly, in step d), the embedded neurosphere can be incubated for 1h to 72h (preferably for 2h to 56h, preferably for 3h to 54h, preferably for 4h to 50h, preferably for 5h to 48h, preferably for 6h to 46h, preferably for 10h to 42h, more preferably for 40h post-embedding. In another embodiment, the embedded neurosphere of step c) incubated for 5h to 55h, preferably for 10h to 50h, more preferably for 15h to 48h, more preferably for 20h to 48h, more preferably for 24h to 40h. It is preferable that the duration of step d) of incubating the embedded neurosphere of step c) is less than 55h, preferably less than 50h, even preferably 48h at the most. This short timing allows to reduce the confounding effect of cell proliferation over cell invasion. Indeed, brain tumour cells (in particular brain tumour stem cells, such GSCs) have a doubling time longer than 48h. Thus, in step d), said next measurement is performed less than 55h, preferably less than 50h, even preferably 48h at the most, after having performed step c). In a particularly preferred embodiment of step d), the embedded neurosphere is incubated for 15h to 25h

Advantageously, in step d), the embedded neurosphere of step c) is incubated under an atmosphere comprising from 16% to 26% oxygen ($O_2$), more preferably from 18% to 24% $O_2$, more preferably from 20% to 22% $O_2$, more preferably approximately 21% $O_2$, even more preferably 21% $O_2$. The atmosphere advantageously further comprises approximately from 2% to 8% carbon dioxide ($CO_2$), more preferably from 4% to 6% $CO_2$, more preferably approximately 5% $CO_2$, even more preferably 5% $CO_2$. Advantageously, the atmosphere further comprises approximately from 80% to 100% humidity, more preferably from 85% to 95% humidity, more preferably approximately 90% humidity, even more preferably 90% humidity.

In step e), it may be advantageous to perform more than one measurement, at different time points. Thus, step e) may be repeated at different time points starting from step c). In such case, step e) preferably comprises measuring the area of the embedded neurosphere after step d), wherein at least two measurement are made at least every 5h from each other, preferably at least every 10h, more preferably at least every 15h, more preferably at least every 20h from each other; preferably using the same technique used in step c); preferably wherein between each measurement, the embedded neurosphere is incubated under the same conditions as in step d); preferably wherein the total incubation time of the embedded neurosphere under the same conditions as in step d), including the total incubation time of step d), is less than 55h, preferably less than 50h, even preferably 48h at the most. In this case, the method of the invention may comprise the further steps g) and h), wherein these steps are as follows:

g) incubating the embedded neurosphere of step e) at a temperature ranging from 35°C to 39°C (preferably at a temperature ranging from 36°C to 38°C, more preferably at a temperature around 37°C, even more preferably at 37°C), for 1h to 72h (preferably for 2h to 56h, preferably for 3h to 54h, preferably for 4h to 50h, preferably for 5h to 48h, preferably for 6h to 46h, preferably for 10h to 42h, more preferably for 40h ; preferably under the same conditions as in step d); preferably wherein the total incubation time of the embedded neurosphere including the total incubation time of step d) is less than 55h, preferably less than 50h, even preferably 48h at the most;

h) measuring the area of the embedded neurosphere after step g), preferably using the same technique used in step c);

i) determining the invasive ability of the brain tumour cells, in particular of the brain tumour stem cells, by comparing the area measured at step c) to the area measured at step h), and/or by comparing the area measured at step e) to the area measured at step h);

j) optionally calculating the mean and/or the median the invasive ability of the brain tumour cells, in particular of the brain tumour stem cells, based on the invasive abilities determined in steps f) and i).

In an advantageous embodiment, step f) comprises calculating the invasion score of the brain tumour cells (in particular of the brain tumour stem cells), wherein the invasion score is the ratio of the area (or the average area, or the median area) of the embedded neurosphere measured at step e) to the area (or the average area, or the median area) of the embedded neurosphere measured at step c) (i.e. the invasion score is the area (or the average area, or the median area) of the embedded neurosphere measured at step e) divided by the area (or the average area, or the median area) of the embedded neurosphere measured at step c)). Advantageously, the invasion score is calculated from the average area, or the median area calculated from at least two neurospheres, by the method described just above. Similarly, further step i) may also comprise calculating the invasion score of the brain tumour cells (in particular of the brain tumour cells), wherein the invasion score is the ratio of the area (or the average area, or the median area) of the embedded neurosphere measured at step h) to the area (or the average area, or the median area) of the embedded neurosphere measured at step c) and/or e).

In a particular embodiment, the higher the invasion score is, the higher is the risk that the brain tumour cells become metastatic and/or have a high invasive ability or metastatic ability.

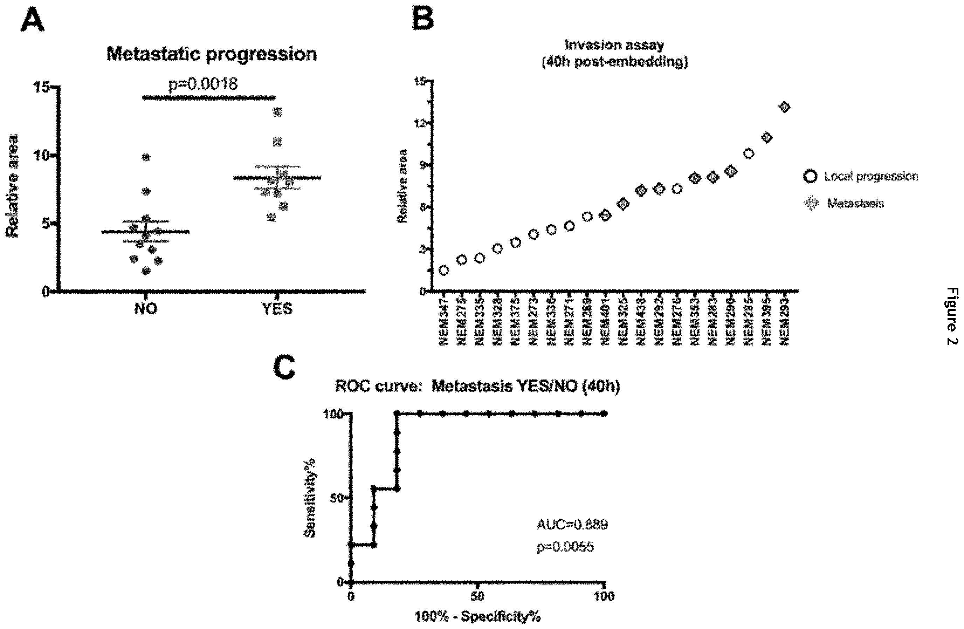

The results obtained by the inventors (Figure 2, Table 1) show that an invasion score inferior to 5.39 indicates that the invasive/metastatic ability of the tested brain tumour cells is null (Table 1, no false negative). Therefore, an invasion score inferior to 5.39 indicates that the invasive/metastatic ability of the tested brain tumour cells is low, preferably null (i.e. the probability that these cells have an invasive or metastatic behaviour is low (e.g. less than 10%), preferably null). Thus, the chances that the tested brain tumour cells have no invasive/metastatic ability are higher than 90%, when the calculated invasion score is lower than 5.39. Consequently, the prognosis of the patient is likely improved when the invasion score calculated with the method of the invention is lower than 5.39.

Conversely, the results of the inventors show that the chances for brain tumour cells (in particular the brain tumour stem cells) having high invasive/metastatic ability to be detected are higher than 75% (preferably higher than 80%), when the calculated invasion score according to the invention is equal to or higher than 5.39 (as demonstrated by Figure 2 and Table 1). Thus, the risk that the tested brain tumour cells have a high invasive ability is higher than 75% (preferably higher than 80%), when the calculated invasion score is equal to or higher than 5.39..

In other words, when the calculated invasion score of the invention is equal to or higher than 5.39 in a subject, the chances to detect actual invasive/metastatic brain tumour cells (in particular brain tumour stem cells) are 75% higher (preferably 80% higher) than in reference cells (preferably non-metastatic brain tumour cells or cells from a reference sample obtained from a non-metastatic brain tumour, or cells from a reference sample with an invasion score below the threshold of 5.39) (see Table 1).

Indeed, the data obtained by the inventors from an initial cohort of 20 primary models showed that an invasion score equal to or higher than 5.39 using the present method for determining the invasive ability on glioma biopsy from glioma patients is strongly correlated with a higher risk of switching to a metastatic glioma form (ability to detect true-positive metastatic events higher than 75%-80%).

Comparing the invasive ability of the brain tumour cells (in particular of the brain tumour stem cells) and/or the invasion score of the brain tumour cells (in particular of the brain tumour stem cells), with the invasive ability of reference brain tumour cells (in particular of reference brain tumour stem cells) and/or the invasion score of reference brain tumour cells (in particular of reference brain tumour stem cells), can be performed by using the *in vitro* method of the invention. Advantageously, the reference brain tumour cells (in particular the reference brain tumour stem cells) are brain tumour cells (in particular reference brain tumour stem cells) at a specific stage (e.g. a benign brain tumour [WHO grade I], or a low-grade brain tumour [WHO grade II], or a high-grade brain tumour [WHO grades III-IV], and/or a metastatic form of brain tumour.

Accordingly, in this embodiment, the *in vitro* method of the invention comprises a further step (e.g. step g') or k)) of comparing the invasive ability of the brain tumour cells (in particular of the brain tumour stem cells) and/or the invasion score of the brain tumour cells (in particular of the brain tumour stem cells), with the invasive ability of the reference brain tumour cells (in particular of the reference brain tumour stem cells) and/or the invasion score of the reference brain tumour cells (in particular of the reference brain tumour stem cells), and optionally, a further step (e.g. step h') or l)) of determining the stage of the brain tumour cells (in particular of the brain tumour stem cells), based on this comparison.

When the invasive ability of glioma cells is determined using the *in vitro* method of the invention, the reference brain tumour cells (in particular the reference brain tumour stem cells) are brain glioma cells (in particular reference GSCs). Advantageously, the reference glioma cells are glioma cells (in particular the reference GSCs) at a specific stage (e.g. a benign glioma [WHO grade I], or a low-grade glioma [WHO grade II], or a high-grade glioma [WHO grades III-IV], and/or a metastatic form of glioma; such as a benign glioblastoma or DMG, or a low-grade glioblastoma or DMG, or a high-grade glioblastoma or DMG, and/or a metastatic form of glioblastoma or DMG). The reference glioma cells preferably comprise(s) GSCs. In an advantageous embodiment, the reference glioma cells (in particular the reference GSCs), have been obtained from a reference tumour sample, wherein the reference tumour sample is preferably a sample from a central nervous system (CNS) tumour, such as a brain tumour sample or a spinal cord tumour sample, more preferably a glioma sample (such as a glioblastoma or a DMG sample), at a specific stage (e.g. a benign glioma [WHO grade I], or a low-grade glioma [WHO grade II], or a high-grade glioma [WHO grades III-IV], and/or a metastatic form of glioma; such as a benign glioblastoma or DMG, or a low-grade glioblastoma or DMG, or a high-grade glioblastoma or DMG, and/or a metastatic form of glioblastoma or DMG).

Accordingly, in this embodiment, the *in vitro* method of the invention comprises a further step (e.g. step g') or k)) of comparing the invasive ability of the glioma cells (in particular of the GSCs) and/or the invasion score of the glioma cells (in particular of the GSCs), with the invasive ability of the reference glioma cells (in particular of the reference GSCs) and/or the invasion score of the reference glioma cells (in particular of the reference GSCs), and optionally, a further step (e.g. step h') or l)) of determining the stage of the glioma cells (in particular of the GSCs), based on this comparison.

The reference glioma cells preferably comprise(s) GSCs. In an advantageous embodiment, the reference glioma cells (in particular the reference GSCs), have been obtained from a reference tumour sample, wherein the reference tumour sample is preferably a sample from a central nervous system (CNS) tumour, such as a brain tumour sample or a spinal cord tumour sample, more preferably a glioma sample (such as a glioblastoma or a DMG sample), at a specific stage (e.g. a benign glioma [WHO grade I], or a low-grade glioma [WHO grade II], or a high-grade glioma [WHO grades III-IV], and/or a metastatic form of glioma; such as a benign glioblastoma or DMG, or a low-grade glioblastoma or DMG, or a high-grade glioblastoma or DMG, and/or a metastatic form of glioblastoma or DMG). In an advantageous embodiment, said reference brain tumour cells (in particular said reference GSCs), such as said reference glioma cells (in particular said reference GSCs), have been obtained from a tumour sample obtained from a reference subject.

Advantageously, the method further comprises the step of obtaining a brain tumour primary cell culture from a tumour sample from a subject prior to step a), wherein the brain tumour primary cell culture and/or the tumour sample preferably comprise(s) brain tumour stem cells , and wherein the tumour sample is preferably a sample from a central nervous system (CNS) tumour, such as a brain tumour sample or a spinal cord tumour sample, more preferably a brain tumour sample (in particular a glioma sample, more particularly a glioblastoma sample or a DMG sample). This prior step can be called step a-0).

Accordingly, the present invention concerns an *in vitro* method for determining the invasive ability of brain tumour cells, in particular brain tumour stem cells, comprising the steps of:

a-0) obtaining a brain tumour primary cell culture from a tumour sample from a subject, wherein the brain tumour primary cell culture and/or the tumour sample preferably comprise(s) brain tumour stem cells; wherein the tumour sample is preferably a sample from a central nervous system (CNS) tumour, such as a brain tumour sample or a spinal cord tumour sample, more preferably a brain tumour sample;

a) obtaining a neurosphere from the brain tumour primary cell culture of step a-0), wherein the neurosphere preferably comprises GSCs;
b) embedding the neurosphere of step a) in a protein-based extracellular-like matrix, preferably using a solution of the protein-based extracellular-like matrix;
c) measuring the area of the embedded neurosphere of step b), preferably using a non-invasive analytical technique, such as an imaging technique;
d) incubating the embedded neurosphere of step c) at a temperature ranging from 35°C to 39°C, (preferably at a temperature ranging from 36°C to 38°C, more preferably at a temperature around 37° C, even more preferably at 37°C), for 1h to 72h (preferably for 2h to 56h, preferably for 3h to 54h, preferably for 4h to 50h, preferably for 5h to 48h, preferably for 6h to 46h, preferably for 10h to 42h, more preferably for 40h);
e) measuring the area of the embedded neurosphere of step d), preferably using the same technique used in step c);

f) Determining the invasive ability of the glioma cells, in particular of the GSCs, by comparing the area measured at step c) to the area measured at step e).

When the *in vitro* method is for determining the invasive ability of glioma cells (in particular GSCs), step a-0) comprises obtaining a glioma primary cell culture from a tumour sample from a subject, wherein the glioma primary cell culture and/or the tumour sample preferably comprise(s) GSCs. In this case, the tumour sample is preferably a glioma sample.

When the *in vitro* method is for determining the invasive ability of glioblastoma cells (in particular glioblastoma stem cells), step a-0) comprises obtaining a glioblastoma primary cell culture from a tumour sample from a subject, wherein the glioblastoma primary cell culture and/or the tumour sample preferably comprise(s) glioblastoma stem cells. In this case, the tumour sample is preferably a glioblastoma sample.

When the *in vitro* method is for determining the invasive ability of DMG cells (in particular DMG stem cells), step a-0) comprises obtaining a DMG primary cell culture from a tumour sample from a subject, wherein the DMG primary cell culture and/or the tumour sample preferably comprise(s) DMG stem cells. In this case, the tumour sample is preferably a DMG sample.

When said subject is a human, it may be an adult or a child.

Advantageously, the step of obtaining a brain tumour primary cell culture (in particular a glioma primary cell culture, more particularly a glioblastoma or a DMG primary cell culture, more particularly a DMG primary cell culture) from a tumour sample from a subject prior to step a) (i.e. step a-0) comprises the following successive phases:

$1_{a-0}$) Dissociating the tumour sample to obtain a cell suspension (preferably a single cell suspension), preferably using a mechanical dissociation technique;

$2_{a-0}$) Growing the single cell suspension of phase $1_{a-0}$) in a serum-free medium adapted to neural stem cells, preferably as adherent monolayers (e.g. on laminin coated cells), at a temperature ranging from 35°C to 39°C (preferably at a temperature ranging from 36°C to 38°C, more preferably at a temperature around 37° C, even more preferably at 37° C), to obtain a brain tumour primary cell culture (in particular a glioma primary cell culture, more particularly a glioblastoma or a DMG primary cell culture, more particularly a DMG primary cell culture); and

$3_{a-0}$) Optionally, passaging the grown cells of phase $2_{a-0}$) when they reach around 65-80% confluence (preferably 65-75% confluence, preferably around 70% confluence), to increase the amount of cells in the primary cell culture, wherein passaging is preferably performed four times or less.

In step $1_{a-0}$), the tumour sample is preferably dissociated in a serum-free medium adapted to neural stem cells.

The serum-free medium adapted to neural stem cells used in phase $1_{a-0}$) and/or the serum-free medium adapted to neural stem cells used in phase $2_{a-0}$) is preferably a serum-free neurobasal stem cell expansion medium (e.g. Neurocult NS-A (for example commercialized by STEMCELL Technologies) supplemented with human-basic FGF (20 ng ml$^{-1}$, for example commercialized by STEMCELL Technologies), human EGF (20 ng ml$^{-1}$, for example commercialized STEMCELL Technologies), PDGF-AA (10 ng ml$^{-1}$, for example commercialized by Miltenyi Biotec), PDGF-BB (10 ng ml$^{-1}$, for example commercialized by Miltenyi Biotec) and heparin (2 $\mu$g ml$^{-1}$, for example commercialized by STEMCELL Technologies).

Advantageously, in phase $2_{a-0}$), the single cell suspension of phase $1_{a-0}$) is grown under an atmosphere comprising from 16% to 26% oxygen ($O_2$), more preferably from 18% to 24% $O_2$, more preferably from 20% to 22% $O_2$, more preferably approximately 21% $O_2$, even more preferably 21% $O_2$. The atmosphere advantageously further comprises approximately from 2% to 8% carbon dioxide ($CO_2$), more preferably from 4% to 6% $CO_2$, more preferably approximately 5% $CO_2$, even more preferably 5% $CO_2$. Advantageously, the atmosphere further comprises approximately from 80% to 100% humidity, more preferably from 85% to 95% humidity, more preferably approximately 90% humidity, even more preferably 90% humidity.

Preferably, the brain tumour cells comprise, or consist essentially of, or are glioma cells (i.e. cells of a glioma) and/or the brain tumour stem cells comprise, or consist essentially of, or are GSCs (i.e. stem cells of a glioma).

Preferably, the glioma cells comprise, or consist essentially of, or are glioblastoma cells (i.e. cells of a glioblastoma) and/or the GSCs comprise, or consist essentially of, or are glioblastoma GSCs (i.e. GSCs of a glioblastoma).

Preferably, the glioma cells comprise, or consist essentially of, or are diffuse midline glioma cells (or DMG cells)(i.e. cells of a DMG) and/or the GSCs comprise, or consist essentially of, or are diffuse midline glioma (DMG) GSCs (i.e. GSCs of a DMG).

In one embodiment, the *in vitro* method for determining the invasive ability of brain tumour cells, in particular brain tumour stem cells, is an *in vitro* method for identifying brain tumour cells having invasive/metastatic abilities, in particular brain tumour stem cells having invasive/metastatic abilities.

*In vitro* **methods for the diagnosis of brain tumour, for the prognosis, for the monitoring, for the stratification, for identifying subjects at risk of metastasis, and/or for predicting the metastasis-associated risk in a**

**subject diagnosed with brain tumour**

The present Inventors have developed an original 3D invasion assay to reliably correlate the invasiveness of biopsy-derived tumour stem cells with the clinical outcome in patients. Using this 3D invasion assay (as described above), the inventors showed a strong correlation between the risk of metastasis in patients and the invasive ability of brain tumour primary cell culture derived from biopsies at diagnosis. Indeed, the Inventors demonstrated that the glioma primary cell culture derived from patients who developed metastases during their progression showed a very high invasiveness, whereas invasiveness from glioma primary cell culture derived from patients without metastatic progression was significantly lower.

In particular, the data surprisingly showed that this assay can be used to rapidly and reliably predict the risk of metastasis development in patients with brain tumour (in particular glioma), with both high specificity and high sensitivity (higher than 80%).

The present invention thus concerns the use of the *in vitro* method (as defined above) for determining the invasive ability of brain tumour cells (in particular glioma cells)for diagnosing an invasive/metastatic brain tumour (in particular an invasive/metastatic glioma), for the prognosis, for the monitoring, for the stratification, for identifying subjects at risk of metastasis, and/or for predicting the metastasis-associated risk in a subject diagnosed with a brain tumour (in particular a glioma).

Accordingly, the present invention concerns an *in vitro* method for diagnosing an invasive/metastatic brain tumour in a subject in need thereof (in particular in a subject already diagnosed with a CNS tumour, such as a brain tumour or a spinal cord tumour, or a subject suspected of having a brain tumour or a spinal cord tumour), comprising the steps of:

a) determining the invasive ability of brain tumour cells, in particular brain tumour stem cells, using the *in vitro* method for determining the invasive ability of brain tumour cells (as defined above); wherein the brain tumour cells, in particular the brain tumour stem cells, have been obtained from a tumour sample from the subject (preferably a brain tumour sample or a spinal cord tumour sample); and
b) diagnosing an invasive/metastatic brain tumour, in the subject based on the invasive ability of step a).

The present invention also concerns an *in vitro* method for diagnosing an invasive/metastatic glioma, in a subject in need thereof (preferably a subject already diagnosed with a CNS tumour, such as a brain tumour or a spinal cord tumour, or a subject suspected of having a brain tumour or a spinal cord tumour), comprising the steps of:

a) determining the invasive ability of glioma cells, in particular glioma stem cells (GSCs), using the *in vitro* method for determining the invasive ability of glioma cells (as defined above); wherein the glioma cells, in particular the GSCs, have been obtained from a tumour sample from the subject (preferably a brain tumour sample or a spinal cord tumour sample, more preferably a glioma sample); and
b) diagnosing an invasive/metastatic glioma in the subject, based on the invasive ability of step a).

The present invention also concerns *in vitro* method for the prognosis, for the monitoring, for the stratification, for identifying subjects at risk of metastasis, and/or for predicting the metastasis-associated risk in a subject diagnosed with a brain tumour (in particular glioma), comprising the steps of:

a) determining the invasive ability of brain tumour cells, in particular brain tumour stem cells (preferably glioma cells, in particular glioma stem cells (GSCs)), using the *in vitro* method for determining the invasive ability of brain tumour cells (as defined above); wherein the brain tumour cells, in particular the brain tumour stem cells, have been obtained from a tumour sample from the subject (preferably from a central nervous system (CNS) tumour, such as a brain tumour sample or a spinal cord tumour sample, more preferably the tumour sample is a glioma sample); and
b) determining the prognosis of the brain tumour (preferably glioma), monitoring the brain tumour (preferably glioma), stratifying the subject diagnosed with a brain tumour (preferably a glioma), identifying the subject at risk of metastasis, and/or predicting the metastasis-associated risk in the subject diagnosed with a brain tumour (preferably a glioma), based on the invasive ability of step a).

Advantageously, the invasion score of the glioma cells (in particular of the GSCs), is determined using the *in vitro* method for determining the invasive ability of glioma cells as defined above.

The data obtained by the inventors showed that an invasion score equal to or higher than 5.39 using the present method is strongly correlated with a high risk of switching to a metastatic glioma form (a risk higher than 75%). Indeed, the ROC analysis on the invasive ability of glioma cells, in particular of the GSCs, showed that both sensitivity and specificity are higher (in particular the specificity is higher than 75%, preferably higher than 80%) when the invasion score obtained is equal to or higher than 5.39 (see table 1). They furthermore showed that when the score is lower than 5.39, no metastatic behaviour is detected (no false negative, see Table 1).

In a preferred embodiment, the subject is diagnosed with invasive and/or metastatic brain tumour, the prognosis of the brain tumour is poor, the brain tumour has worsened (in particular the brain tumour has more than 75% of risk to become metastatic, preferably more than 80% of risk), the subject is stratified and/or identified as a subject at risk of metastasis (in particular at having 75% risk of metastasis, preferably more than 80% risk), and/or the metastasis-associated risk in the subject is high (in particular higher than 75%, preferably more than 80%), when the invasion score of the brain tumour cells (in particular of the brain tumour stem cells) as defined above is equal to or higher than 5.39.

Conversely, in another preferred embodiment, the subject is diagnosed with non-invasive and/or metastatic brain tumour, the prognosis of the brain tumour is improved, the brain tumour will not worsen (or the brain tumour has no risk to become metastatic), the subject is stratified and/or identified as a subject having no risk of metastasis, and/or the metastasis-associated risk in the subject is very low (in particular lower than 10%, preferably null), when the invasion score of the brain tumour cells (in particular of the brain tumour stem cells) as defined above is inferior to 5.39.

In a preferred embodiment, the subject is diagnosed with invasive and/or metastatic glioma, the prognosis of the glioma is poor, the glioma has worsened (in particular the glioma has more than 75% of risk to become metastatic, preferably more than 80% of risk), the subject is stratified and/or identified as a subject at risk of metastasis (in particular at having 75% risk of metastasis, preferably 80% risk), when the invasion score of the glioma cells (in particular of the GSCs) as defined above is equal to or higher than 5.39.

Conversely, in another preferred embodiment, the subject is diagnosed with non-invasive and/or metastatic glioma, the prognosis of the glioma improved, the glioma will not worsen (or the glioma has no risk to become metastatic), the subject is stratified and/or identified as a subject having no risk of metastasis, and/or the metastasis-associated risk in the subject is very low (in particular lower than 10%, preferably null), when the invasion score of the glioma cells (in particular of the GSCs) as defined above is inferior to 5.39.

Preferably, the subject has been diagnosed with glioblastoma or DMG, more preferably diffuse midline glioma (DMG). Thus, in this embodiment, the glioma is a glioblastoma or a DMG, more preferably a DMG.

In an advantageous embodiment, the present method is for selecting a therapy for treating said subject, or for assessing the efficacy of a therapy in a treated subject, or for adapting a therapy in a treated subject.

**Uses as biomarkers**

The Inventors have shown that the invasive ability of brain tumour cells (in particular the invasion score of brain tumour cells) can be used as a biomarker for the prognosis, for the monitoring, for the stratification, for determining the outcome, or any combination thereof, of a brain tumour (such as a glioma, in particular a glioblastoma or a DMG). The invasive ability of brain tumour cells (in particular the invasion score of brain tumour cells) can also be used as a biomarker for identifying subjects at risk of metastasis, and/or for predicting the metastasis-associated risk in a subject diagnosed with brain tumour. The data also suggest that the invasive ability of brain tumour cells (in particular the invasion score of brain tumour cells) are useful for selecting a therapy for a subject suffering from a brain tumour, for assessing the efficacy of a therapy in a subject suffering from a brain tumour and for adapting a brain tumour therapy for a brain tumour-suffering subject.

The present invention thus relates to the use of the invasive ability of brain tumour cells (in particular the invasion score of brain tumour cells) as a biomarker of metastasis in a brain tumour, of a subject.

The present invention thus also relates to the use of the invasive ability of glioma cells (in particular the invasion score of glioma cells) as a biomarker of metastasis in a glioma, of a subject.

More precisely, these biomarkers can be used for the prognosis, for the monitoring, for the stratification, or for determining the outcome of a brain tumour (in particular a glioma, more particularly a glioblastoma or a DMG) in a subject; and/or for assessing the risk, for a subject suffering from a brain tumour (in particular a glioma, more particularly a glioblastoma or a DMG) of switching to a metastatic form of glioma and/or for identifying a subject at risk of metastasis; and/or for selecting a therapy for a subject suffering from a brain tumour (in particular a glioma, more particularly a glioblastoma or a DMG); and/or for assessing the efficacy of a therapy in a subject suffering from a brain tumour (in particular a glioma, more particularly a glioblastoma or a DMG); and/or for adapting a brain tumour- (in particular a glioma-, more particularly a glioblastoma- or a DMG-) therapy.

**DESCRIPTION OF THE FIGURES**

**Figure 1:** Protein-based extracellular-like matrix (Matrigel®) invasion assay and quantification. Example of imaging and quantification of the area of one neurosphere immediately after embedding in the extracellular-like matrix (A), 24 hours (B) and 40 hours (C) post-embedding. The considered area of the sphere is outlined in grey, post-acquisition.

Only cells in physical contact with the structure are considered within the invasion area, whereas single cells or cluster are excluded. Images were acquired using an EVOS m5000 microscope (Thermo Fisher) equipped with a long working distance 4X objective and high-resolution camera. Scale bars correspond to 600 $\mu$m.

**Figure 2:** Protein-based extracellular-like matrix (Matrigel®) invasion assay and prediction of metastasis risk. Panel A shows the difference between the invasiveness of biopsy-derived cells in 20 patients with and without metastasis during the course of the disease. Panel B shows the dispersion of the same results: the models with a high invasion coefficient are mostly those derived from patients who developed metastasis later. Panel C represents the results of a Receiver Operating Characteristic (ROC) analysis. This analysis is used to guide the observer in choosing a threshold that represents the best compromise between sensitivity and specificity of the analysis. Area under the curve (AUC) indicates a good (close to excellent) accuracy of the test in predicting metastatic relapse.

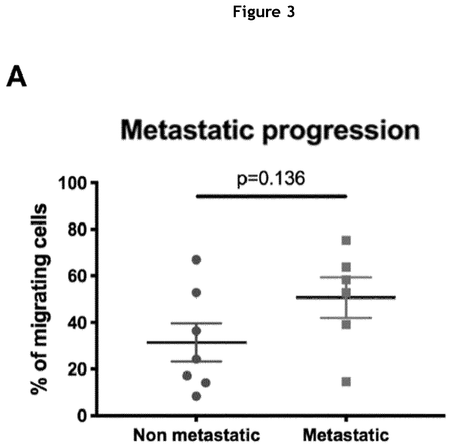

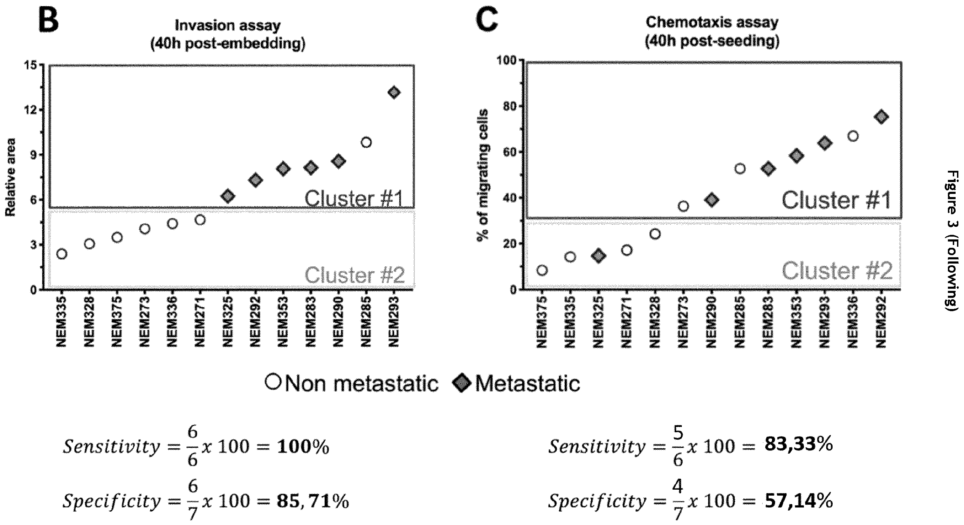

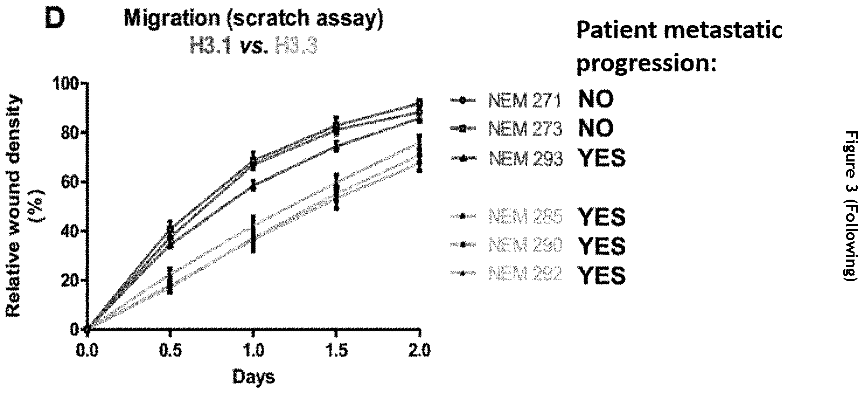

**Figure 3:** Comparison of three methodologies for evaluating the metastatic risk with biopsy-derived models at diagnosis. Panel A shows the percentage of migrating cells 40 hours after seeding in biopsy-derived cell models from 13 patients with and without metastasis during the course of the disease. Panel B shows the dispersion of data obtained using a protein-based extracellular-like matrix (Matrigel®) invasion assay in a cohort of 13 patient-derived models 40 hours post-embedding; threshold value defining the two groups (high and low invasion) was determined using a k-means analysis. Panel C shows the dispersion of migration rate (as a percentage of migrating cells 40 hours post seeding) obtained using a boyden-like migration assay for the same 13 patient-derived cell models; threshold value defining the two groups (high and low invasion) was determined using a k-means analysis. Panel D: Scratch-wound assay and metastasis risk. The graph shows dynamic of wound recovering (as a percentage of cell density) within 48 hours after seeding in biopsy-derived cell models from 6 patients with and without metastasis during the course of the disease. The results shown the lack of any correlation between cell migration as measured with this assay and clinical progression in those patients.

## EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

**EXAMPLE** :

### 1. Materials and Methods

#### 1.1. General presentation of the Method

The present Inventors have developed an original three-dimensional invasion assay that can be used to efficiently and accurately determine and measure the invasive ability of brain tumour cells, in particular glioma cells, more particularly glioma stem cells (GSCs). This innovative invasion assay has been successfully used on GSCs directly derived from stereotactic biopsies collected from patients at diagnosis. The results from this assay show a correlation with the risk of metastatic progression in DIPG patients for whom primary GSCs model were available.

Briefly, primary models of tumour stem cells were isolated from biopsies obtained by stereotactic biopsies performed at the Necker-Enfant Malades Hospital and cultured in a medium suitable for neural stem cell maintenance (Neurocult complete with bFGF, EGF and PDGF). The cultures were used at early passaging (p1-p4) for the formation of neurospheres for 72 hours. Those neurospheres were embedded in a protein extracellular-like matrix (e.g. Matrigel® at an initial concentration > 10mg/ml), and the area of invasion was measured by microscopy at different times. The invasion score at 40h post-inclusion, corresponding to the ratio between the surface area at 40h and the surface area of the sphere after inclusion (at the initial time), was correlated with the clinical data obtained in the patients.

#### 1.2. Establishment and propagation of GSC primary models from patients:

Tissue collection from newly-diagnosed patients was systematically performed by trained neuro-surgeons in Necker-Enfants Malades hospital (Paris, France) under informed consent. Tumour biopsy was preserved in DMEM with 100U ml-1 penicillin and 100 $\mu$g ml-1 of streptomycin (GIBCO).

Within 24 hours post-surgery, tumours were mechanically dissociated by vigorous pipetting into serum-free medium to obtain a single cell suspension.

Culture flasks or dishes were laminin-coated (Invitrogen) at least 3 hours before cell plating. Cells were then cultured in these supports as an adherent monolayer using a NeuroCult NS-A medium with proliferation supplement (STEMCELL Technologies), also supplemented with human-basic FGF (20 ng ml-1, STEMCELL Technologies), human EGF (20 ng ml-1, STEMCELL Technologies), PDGF-AA (10 ng ml-1, Miltenyi Biotec), PDGF-BB (10 ng ml-1, Miltenyi Biotec) and heparin (2 μg ml-1, STEMCELL Technologies).

Medium was completely renewed every 2-3 days and passaging performed when cells were at 70% confluence, using Accutase (GIBCO) to detach cells. Alternatively, cells can be frozen gradually in complete medium supplemented with 10% dimethyl sulfoxide (DMSO, Sigma-Aldrich).

### 1.3. Neurosphere formation and transferring to Matrigel® extracellular matrix droplets

Invasion assay was performed on patient-derived GSC models at passaging 1-4, using Matrigel® basal matrix (Corning) at initial concentration superior to 10mg/ml of protein.

Cells were detached with Accutase, and counted manually using a hemocytometer.

A volume of cells suspension equivalent to 20.000 cell per experimental replicate is centrifuged 400g and resuspended in a volume equivalent to 100 μl of complete medium per experimental replicates. A volume of 100 μl of cells suspension (corresponding to 20.000 cells) is then dispatched into at least 24 wells in a U-bottom ultra-low attachment 96-wells plate (Corning). It is preferred that cells do not adhere to plastic.

Cells were let form neurospheres during 48-72 hours, until the spheres appear round-shaped with smooth edges.

The neurospheres are centrifuged at 200g (unity of relative centrifugal force, RCF) to remove excess of medium, and resuspended in 5μl of fresh complete medium. A volume of 25μl of ice-cold Matrigel® is added at a concentration of at least 10mg/ml. The neurosphere-containing Matrigel® droplets are incubated at 37°C for 25 min, to let the Matrigel® polymerize.

By using surgical forceps, each Matrigel® droplet containing a neurosphere is carefully transferred into a well of a 24-well containing 1ml of complete medium. Image acquiring is performed immediately after.

### 1.4. Imaging and post-acquisition analysis.

Size measurement was performed using an inverted microscope equipped with a camera at 5x magnification. Initial images were acquired immediately after neurosphere embedding in Matrigel®, and then 24 and 40 hours after. This short timing is intended to reduce the confounding effect of cell proliferation over invasion, since all GSC models analysed have a doubling-time longer than 48 hours (10).

Once collection of images is complete for a given patient-derived model, images are analysed using a measurement software (e.g. Fiji) to determine the area of the neurosphere, just after embedding in Matrigel and at later time points (Figure 1 shows examples of such acquired images). Quantification is performed on multiple technical replicates to reduce experimental variation. Invasive ability at a given time point is determined as a ratio representing the average relative neurosphere area.

The invasion score is calculated with the following formula:

$$\text{Invasion score} = (\text{Area at 24 or 40 hours})/(\text{Area at 0 hours})$$

The *in vitro* results are then compared to the clinical data available for the patients.

### 1.6. Receiver Operating Characteristic (ROC)

Analysis was performed using Prism GraphPad statistics software, version 7a. This type of curve plays a central role in evaluating the ability of tests to discriminate the true state of subjects, finding the optimal cut off values. The area under the curve (AUC) represents a measure of the accuracy, frequently used for evaluating diagnostic test (16).

### 1.7. Chemotaxis (Boyden-like) assay (Technique of the prior art)

Both top and bottom parts of specific ClearView 96-well plates (4582, EssenBioscience) were coated with laminin (Thermo Fisher scientific). Primary gliomas stem cells (GSCs) prepared as in 1.2. were seeded at 1,000-2,000 cells per well in the top plate in presence of Neurocult complete medium deprived of growth factors. Cells were allowed to settle down for 30 minutes. Then the top plate was inserted onto the bottom reservoir plate containing complete Neurocult medium with growth factors (EGF, FGF, and PDGF). As an experimental negative control, the reservoir plate was filled with medium deprived of growth factors. Finally, cell migration was recorded by video-microscopy using a Zoom IncuCyte (EssenBioscience) for three days. Post-treatment analysis of acquired images was performed using the Incucyte analysis software (IncuCyte Zoom 2015A, EssenBioscience).

### 1.8. Scratch-wound assay (Technique of the prior art)

96-well plates were coated with laminin. GSC were seeded at a density ranging from 50,000 to 66,000 cells/well. After seeding, cells were allowed to settle down and attach for 4 hours at 37°C in an incubator at 20% oxygen. Then, plates were placed into the wound maker tool (EssenBioscience) and the scratch made using the 96-well comb. Wells were washed with cold D-PBS to remove debris and 100 μl of medium containing growth factors (EGF, FGF, and PDGF) was added to each well. The wound healing closure was recorded by video-microscopy (IncuCyte Zoom, EssenBioscience) for 3 days and the post-treatment of acquired images was performed using the software provided by the manufacturer (IncuCyte Zoom 2015A, EssenBioscience). Percentage of migration was analyzed by the percentage of confluence of mask of cells.

### 2. Results

A cohort of 20 patients was used for the Protein-based extracellular-like matrix (Matrigel®) invasion assay. Results show that the invasion score at 40h post-inclusion, corresponding to the ratio between the surface area at 40h and the surface area of the neurosphere after inclusion (at the initial time), was correlated with the clinical data obtained in the patients (Figure 2). The invasion area is significantly higher in models derived from patients with metastasis than in patients without metastasis (Figure 2A). The models with a high invasion coefficient are mostly those derived from patients who developed metastasis later (Figure 2B).

Table 1 and Figure 2C represent the results of a Receiver Operating Characteristic (ROC) analysis. Accuracy of the test measured by the area under the ROC curve (AUC) was 0.889, where an AUC=1 represents a perfect test, whereas an AUC=0.5 represents a worthless test. This analysis is used to guide the observer in choosing a threshold that represents the best compromise between sensitivity and specificity of the analysis.

*Table 1: sensitivity/specificity table generated using Prism-Graphpad 7.*

| Invasion score | Sensitivity(%) | 95% CI | Specificity(%) | 95% CI | Likelihood ratio |
|---|---|---|---|---|---|
| > 1.882 | 100 | 66,37% to 100% | 9,091 | 0,2299% to 41,28% | 1,1 |
| > 2.326 | 100 | 66,37% to 100% | 18,18 | 2,283% to 51,78% | 1,222 |
| > 2.726 | 100 | 66,37% to 100% | 27,27 | 6,022% to 60,97% | 1,375 |
| > 3.279 | 100 | 66,37% to 100% | 36,36 | 10,93% to 69,21% | 1,571 |
| > 3.783 | 100 | 66,37% to 100% | 45,45 | 16,75% to 76,62% | 1,833 |
| > 4.241 | 100 | 66,37% to 100% | 54,55 | 23,38% to 83,25% | 2,2 |
| > 4.541 | 100 | 66,37% to 100% | 63,64 | 30,79% to 89,07% | 2,75 |
| > 5.007 | 100 | 66,37% to 100% | 72,73 | 39,03% to 93,98% | 3,667 |
| > 5.39 | 100 | 66,37% to 100% | 81,82 | 48,22% to 97,72% | 5,5 |
| > 5.838 | 88,89 | 51,75% to 99,72% | 81,82 | 48,22% to 97,72% | 4,889 |
| > 6.721 | 77,78 | 39,99% to 97,19% | 81,82 | 48,22% to 97,72% | 4,278 |
| > 7.257 | 66,67 | 29,93% to 92,51% | 81,82 | 48,22% to 97,72% | 3,667 |
| > 7.315 | 55,56 | 21,2% to 86,3% | 81,82 | 48,22% to 97,72% | 3,056 |
| > 7.694 | 55,56 | 21,2% to 86,3% | 90,91 | 58,72% to 99,77% | 6,111 |
| > 8.107 | 44,44 | 13,7% to 78,8% | 90,91 | 58,72% to 99,77% | 4,889 |
| > 8.355 | 33,33 | 7,485% to 70,07% | 90,91 | 58,72% to 99,77% | 3,667 |
| > 9.201 | 22,22 | 2,814% to 60,01% | 90,91 | 58,72% to 99,77% | 2,444 |
| > 10.41 | 22,22 | 2,814% to 60,01% | 100 | 71,51% to 100% | |

(continued)

| Invasion score | Sensitivity(%) | 95% CI | Specificity(%) | 95% CI | Likelihood ratio |
|---|---|---|---|---|---|
| > 12.07 | 11,11 | 0,2809% to 48,25% | 100 | 71,51% to 100% | |

Quantification showed a strong heterogeneity in invasiveness (invasion score between 2.4 and 13.2) and a remarkable correlation between *in vitro* data and clinical profiles. In particular, models derived from patients who developed metastases during their progression showed a very high invasiveness and significantly different from models derived from patients without metastatic progression. For a representative cohort of 20 primary models, a ROC analysis showed a sensitivity (percentage of true positives) of 100% and a specificity (percentage of true negatives) of 81,8%.

Indeed, table 1 shows that by imposing a minimum threshold of 5.39 it is possible to predict the totality (9/9) of patients who have developed metastasis, with a specificity >80% (in the present cohort, only two out of eleven patients who have not developed metastasis would be considered at risk).

To confirm that this assay is more sensitive and more specific in predicting metastasis risk than the tests classically used for determining cell migration or invasion abilities (Chemotaxis assay, also called Boyden-like assay, and scratch-wound assay), the results obtained with these 3 different assays have been compared for a cohort of 13 patients. Figure 3 shows that the invasion assay (Figure 3B) provided a higher correlation between cell invasion/migration and clinical progression in those patients than the boyden-like migration assay (Figure 3C) and scratch-wound assay (Figure 3D). For both boyden-like migration assay and scratch-wound assay, sensitivity and specificity of the technique in detecting risk of metastasis were significantly reduced compared to extracellular matrix-like invasion test.

### 3. Discussion

The data show that the 3D invasion assay developed by the Inventors is an efficient, reliable and rapid tool for the prediction of the risk of metastasis development in patients with midline infiltrating gliomas (MIG). The time to obtain an invasion score with this method can vary between 3 and 6 weeks from biopsy receipt due to the highly variable growth rate of the cell models after culture. This delay is compatible with the adaptation of the treatment regimen. The analyses suggest that an invasion score higher than the threshold determined by a Receiver Operating Characteristic (ROC) test in the *in vitro* assay performed under the conditions described in the protocol indicates a high risk (sensitivity close to 100%, specificity higher than 80%) for the patient to develop one or more metastases upon relapse.

### BIBLIOGRAPHIC REFERENCES

1. Sturm D, Witt H, Hovestadt V, Khuong-Quang D-A, Jones DTW, Konermann C, et al. Hotspot Mutations in H3F3A and IDH1 Define Distinct Epigenetic and Biological Subgroups of Glioblastoma. Cancer Cell. 2012;22:425-37.

2. Louis DN, Perry A, Reifenberger G, von Deimling A, Figarella-Branger D, Cavenee WK, et al. The 2016 World Health Organization Classification of Tumors of the Central Nervous System: a summary. Acta Neuropathol. 2016;131:803-20.

3. Chen R, Smith-Cohn M, Cohen AL, Colman H. Glioma Subclassifications and Their Clinical Significance. Neurotherapeutics. 2017;14:284-97.

4. Caretti V, Bugiani M, Freret M, Schellen P, Jansen M, van Vuurden D, et al. Subventricular spread of diffuse intrinsic pontine glioma. Acta Neuropathol. 2014;128:605-7.

5. Castel D, Philippe C, Calmon R, Le Dret L, Truffaux N, Boddaert N, et al. Histone H3F3A and HIST1H3B K27M mutations define two subgroups of diffuse intrinsic pontine gliomas with different prognosis and phenotypes. Acta Neuropathol. 2015;130:815-27.

6. Werbrouck C, Evangelista CCS, Lobón-Iglesias M-J, Barret E, Le Teuff G, Merlevede J, et al. TP53 pathway alterations drive radioresistance in Diffuse Intrinsic Pontine Gliomas (DIPG). Clin Cancer Res. 2019;

7. Kluiver TA, Alieva M, van Vuurden DG, Wehrens EJ, Rios AC. Invaders Exposed: Understanding and Targeting Tumor Cell Invasion in Diffuse Intrinsic Pontine Glioma. Front Oncol. 2020;10:92.

8. Liang C-C, Park AY, Guan J-L. In vitro scratch assay: a convenient and inexpensive method for analysis of cell migration in vitro. Nat Protoc. 2007;2:329-33.

9. Guy J-B, Espenel S, Vallard A, Battiston-Montagne P, Wozny A-S, Ardail D, et al. Evaluation of the Cell Invasion and Migration Process: A Comparison of the Video Microscope-based Scratch Wound Assay and the Boyden Chamber Assay. J Vis Exp [Internet]. 2017 [cited 2020 Jul 29]; Available from: https://www.ncbi.nlm.nih.gov/pmc/articles/PMC5755419/

10. Plessier A, Le Dret L, Varlet P, Beccaria K, Lacombe J, Mériaux S, et al. New in vivo avatars of diffuse intrinsic pontine gliomas (DIPG) from stereotactic biopsies performed at diagnosis. Oncotarget. 2017;8:52543-59.

11. Reynolds BA, Weiss S. Generation of neurons and astrocytes from isolated cells of the adult mammalian central nervous system. Science. American Association for the Advancement of Science; 1992;255:1707-10.

12. Vinci M, Molinari V, Taylor K, Carvalho D, Burford A, Mackay A, et al. HG-99A PATIENT-DERIVED PAEDIATRIC HIGH GRADE GLIOMA AND DIPG CELL CULTURE PANEL RECAPITULATING THE GENOTYPIC AND PHENOTYPIC DIVERSITY OF THE DISEASE. Neuro Oncol. 2016;18:iii71.

13. Vinci M, Burford A, Molinari V, Kessler K, Popov S, Clarke M, et al. Functional diversity and cooperativity between subclonal populations of pediatric glioblastoma and diffuse intrinsic pontine glioma cells. Nat Med. 2018;24:1204-15.

14. Pollard SM. In vitro expansion of fetal neural progenitors as adherent cell lines. Methods Mol Biol. 2013;1059:13-24.

15. Pollard SM, Yoshikawa K, Clarke ID, Danovi D, Stricker S, Russell R, et al. Glioma Stem Cell Lines Expanded in Adherent Culture Have Tumor-Specific Phenotypes and Are Suitable for Chemical and Genetic Screens. Cell Stem Cell. 2009;4:568-80.

16. Hajian-Tilaki K. Receiver Operating Characteristic (ROC) Curve Analysis for Medical Diagnostic Test Evaluation. Caspian J Intern Med. 2013;4:627-35.

## Claims

1. An *in vitro* method for determining the invasive ability of brain tumour cells, in particular brain tumour stem cells, comprising the steps of:

   a) obtaining a neurosphere from a brain tumour primary cell culture,
   b) embedding the neurosphere of step a) in a protein-based extracellular-like matrix,
   c) measuring the area of the embedded neurosphere of step b), preferably using a non-invasive analytical technique;
   d) incubating the embedded neurosphere of step c) at a temperature ranging from 35°C to 39°C, for 1h to 72h;
   e) measuring the area of the embedded neurosphere after step d), preferably using the same technique used in step c);
   f) determining the invasive ability of the brain tumour cells by comparing the area measured at step c) to the area measured at step e).

2. The method of claim 1, wherein the protein-based extracellular-like matrix comprises at least one basement membrane component, wherein the basement membrane component is preferably selected from the group consisting of :

   - Laminin;
   - Collagen,
   - Nidogen, and
   - Any combination thereof,

   and wherein the protein-based extracellular-like matrix optionally further comprises at least one of the following components:

- at least one growth factor, preferably selected from the group consisting of: Transforming Growth Factor Beta (TGF-beta), Epidermal Growth Factor (EGF), Insulin-like Growth Factor 1 (IGF-1), Basic Fibroblast Growth Factor (bFGF), Tissue Plasminogen Activator, Platelet Derived Growth Factor (PDGF), and any combination thereof;
- Heparan sulfate proteoglycan.

3. The method of claim 1 or 2, wherein the solution of the protein-based extracellular-like matrix comprises at least 5mg/mL of proteins.

4. The method of any one of the preceding claims, wherein step b) of embedding comprises two steps b1) and b2), wherein step b1) comprises incorporating the neurosphere in a solution of the protein-based extracellular-like matrix ; and step b2) comprises incubating said solution of step b1) at a temperature ranging from 35°C to 39°C, until the protein-based extracellular-like matrix solution is polymerized to form the protein-based matrix.

5. The method of any one of the preceding claims, wherein the solution of the protein-based extracellular-like matrix comprises a basement component extract, preferably a basement component extract obtained from an Engelbreth-Holm-Swarm (EHS) mouse sarcoma.

6. The method of any one of the preceding claims, further comprising the step of obtaining a brain tumour primary cell culture from a tumour sample from a subject prior to step a).

7. The method of any one of the preceding claims, wherein the brain tumour cells are glioma cells, preferably glioblastoma cells or diffuse midline glioma cells (DMG cells).

8. The method of any one of the preceding claims, wherein step f) comprises calculating the invasion score of the brain tumour cells, wherein the invasion score is the ratio of the area of the embedded neurosphere measured at step e) to the area of the embedded neurosphere measured at step c).

9. The method of claim 8, wherein the risk that the brain tumour cells have a high invasive ability is higher than 75%, when the calculated invasion score is equal to or higher than 5.39.

10. The method of claim 8 or 9, wherein the chances that the brain tumour cells have no invasive/metastatic ability are higher than 90%, when the calculated invasion score is lower than 5.39.

11. An *in vitro* method for diagnosing a metastatic brain tumour in a subject and/or for the prognosis, for the monitoring, for the stratification, for identifying subjects at risk of metastasis, and/or for predicting the metastasis-associated risk in a subject diagnosed with a brain tumour, comprising the step of determining the invasive ability of brain tumour cells using the *in vitro* method according to any one of claims 1 to 10.

12. The method of claim 11, wherein an invasion score of the brain tumour cells is determined by using the method of any one of claims 9 to 10.

13. The method of claim 12, wherein the subject is diagnosed with a metastatic brain tumour, and/or the prognosis of the brain tumour is poor, the brain tumour has worsened, the subject is stratified and/or identified as a subject at risk of metastasis, and/or the metastasis-associated risk in the subject is high, when the invasion score of the glioma cells is equal to or higher than 5.39.

14. The method of any one of claims 11 to 13, wherein the subject has been diagnosed with glioma, preferably glioblastoma or diffuse midline glioma (DMG).

15. The method of any one of claims 11 to 14, for selecting a therapy for treating said subject, or for assessing the efficacy of a therapy in a treated subject, or for adapting a therapy in a treated subject.

## Figure 1

Figure 2

Figure 3

**A**

## Metastatic progression

**B** Invasion assay (40h post-embedding)

**C** Chemotaxis assay (40h post-seeding)

○ Non metastatic ◆ Metastatic

$$Sensitivity = \frac{6}{6} x\ 100 = \mathbf{100}\%$$

$$Specificity = \frac{6}{7} x\ 100 = \mathbf{85,71}\%$$

$$Sensitivity = \frac{5}{6} x\ 100 = \mathbf{83,33}\%$$

$$Specificity = \frac{4}{7} x\ 100 = \mathbf{57,14}\%$$

EP 3 968 025 A1

Figure 3 (Following)

**D** Migration (scratch assay) H3.1 *vs.* H3.3

Patient metastatic progression:

NEM 271 **NO**
NEM 273 **NO**
NEM 293 **YES**

NEM 285 **YES**
NEM 290 **YES**
NEM 292 **YES**

Figure 3 (Following)

Europäisches
Patentamt

European
Patent Office

Office européen
des brevets

## EUROPEAN SEARCH REPORT

Application Number

EP 20 30 6031

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TAMAKI MASASHI ET AL: "IMPLANTATION OF C6 ASTROCYTOMA SPHEROID INTO COLLAGEN TYPE I GELS: INVASIVE, PROLIFERATIVE, AND ENZYMATIC CHARACTERIZATIONS", JOURNAL OF NEUROSURGERY, AMERICAN ASSOCIATION OF NEUROLOGICAL SURGEONS, US, vol. 87, no. 4, 1 January 1997 (1997-01-01), pages 602-609, XP009077270, ISSN: 0022-3085 | 1,3-6,8, 11 | INV. G01N33/574 |
| A | * abstract; figures 1-7 * | 9,10 | |
| X | WO 2017/035315 A1 (UNIV JOHNS HOPKINS [US]) 2 March 2017 (2017-03-02) * paragraph [0053]; examples 1-4 * | 1,2,4-7, 11-15 | |
| T | L De Ridder ET AL: "Autologous spheroid culture: a screening tool for human brain tumour invasion", Critical Reviews in Oncology and Hematology, 1 January 2000 (2000-01-01), pages 107-122, XP055710063, Netherlands DOI: 10.1016/S1040-8428(00)00081-0 Retrieved from the Internet: URL:https://www.sciencedirect.com/science/article/pii/S1040842800000810?via%3Dihub | | TECHNICAL FIELDS SEARCHED (IPC) G01N |
| T | DANNY DEL DUCA ET AL: "Spheroid preparation from hanging drops: characterization of a model of brain tumor invasion", JOURNAL OF NEURO-ONCOLOGY, KLUWER ACADEMIC PUBLISHERS, BO, vol. 67, 1 January 2004 (2004-01-01), pages 295-303, XP007913187, ISSN: 1573-7373 | | |

-/--

The present search report has been drawn up for all claims

| Place of search | Date of completion of the search | Examiner |
|---|---|---|
| The Hague | 11 February 2021 | Rosin, Oliver |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 4

Europäisches
Patentamt

European
Patent Office

Office européen
des brevets

# EUROPEAN SEARCH REPORT

Application Number

EP 20 30 6031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | SARKAR SUSOBHAN ET AL: "Reduction of protein kinase C delta attenuates tenascin-C stimulated glioma invasion in three-dimensional matrix", CARCINOGENESIS., vol. 31, no. 2, 4 December 2009 (2009-12-04), pages 311-317, XP055774507, GB ISSN: 0143-3334, DOI: 10.1093/carcin/bgp297 ----- | | |
| T | FLORCZYK STEPHEN J ET AL: "Porous chitosan-hyaluronic acid scaffolds as a mimic of glioblastoma microenvironment ECM", BIOMATERIALS, ELSEVIER, AMSTERDAM, NL, vol. 34, no. 38, 26 September 2013 (2013-09-26), pages 10143-10150, XP028735923, ISSN: 0142-9612, DOI: 10.1016/J.BIOMATERIALS.2013.09.034 ----- | | TECHNICAL FIELDS SEARCHED (IPC) |
| T | BADRIPRASAD ANANTHANARAYANAN ET AL: "Elucidating the mechanobiology of malignant brain tumors using a brain matrix-mimetic hyaluronic acid hydrogel platform", BIOMATERIALS, ELSEVIER, AMSTERDAM, NL, vol. 32, no. 31, 4 July 2011 (2011-07-04), pages 7913-7923, XP028268208, ISSN: 0142-9612, DOI: 10.1016/J.BIOMATERIALS.2011.07.005 [retrieved on 2011-07-07] ----- -/-- | | |

The present search report has been drawn up for all claims

| Place of search | Date of completion of the search | Examiner |
|---|---|---|
| The Hague | 11 February 2021 | Rosin, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................

& : member of the same patent family, corresponding document

EPO FORM 1503 03.82 (P04C01)

page 2 of 4

Europäisches Patentamt

European Patent Office

Office européen des brevets

**EUROPEAN SEARCH REPORT**

Application Number

EP 20 30 6031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | DAVID L. ET AL: "Collagens, stromal cell-derived factor-1[alpha] and basic fibroblast growth factor increase cancer cell invasiveness in a hyaluronan hydrogel", CELL PROLIFERATION, vol. 41, no. 2, 1 April 2008 (2008-04-01), pages 348-364, XP055774515, GB ISSN: 0960-7722, DOI: 10.1111/j.1365-2184.2008.00515.x ----- | | |
| T | VAJKOCZY PETER ET AL: "Targeting angiogenesis inhibits tumor infiltration and expression of the pro-invasive protein SPARC", INTERNATIONAL JOURNAL OF CANCER, vol. 87, no. 2, 15 July 2000 (2000-07-15), pages 261-268, XP055774518, US ISSN: 0020-7136, DOI: 10.1002/1097-0215(20000715)87:2<261::AID-IJC18>3.0.CO;2-6 ----- | | |
| T | GUILLEM RAMIS ET AL: "EGFR Inhibition in Glioma Cells Modulates Rho Signaling to Inhibit Cell Motility and Invasion and Cooperates with Temozolomide to Reduce Cell Growth", PLOS ONE, vol. 7, no. 6, 6 June 2012 (2012-06-06), page e38770, XP055397901, DOI: 10.1371/journal.pone.0038770 ----- -/-- | | TECHNICAL FIELDS SEARCHED (IPC) |

The present search report has been drawn up for all claims

| Place of search | Date of completion of the search | Examiner |
|---|---|---|
| The Hague | 11 February 2021 | Rosin, Oliver |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 3 of 4

32

Europäisches
Patentamt

European
Patent Office

Office européen
des brevets

# EUROPEAN SEARCH REPORT

Application Number

EP 20 30 6031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | HARADA TAKAMASA ET AL: "Nuclear lamin stiffness is a barrier to 3D-migration, but softness can limit survival", 2014 40TH ANNUAL NORTHEAST BIOENGINEERING CONFERENCE (NEBEC), IEEE, 25 April 2014 (2014-04-25), pages 1-2, XP032692820, DOI: 10.1109/NEBEC.2014.6972810 [retrieved on 2014-12-02] ----- | | |

TECHNICAL FIELDS
SEARCHED          (IPC)

The present search report has been drawn up for all claims

| Place of search | Date of completion of the search | Examiner |
|---|---|---|
| The Hague | 11 February 2021 | Rosin, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 4

**EP 3 968 025 A1**

## ANNEX TO THE EUROPEAN SEARCH REPORT
## ON EUROPEAN PATENT APPLICATION NO.

EP 20 30 6031

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2021

| Patent document cited in search report | Publication date | Patent family member(s) | Publication date |
|---|---|---|---|
| WO 2017035315 A1 | 02-03-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **STURM D ; WITT H ; HOVESTADT V ; KHUONG-QUANG D-A ; JONES DTW ; KONERMANN C et al.** Hotspot Mutations in H3F3A and IDH1 Define Distinct Epigenetic and Biological Subgroups of Glioblastoma. *Cancer Cell,* 2012, vol. 22, 425-37
- **LOUIS DN ; PERRY A ; REIFENBERGER G ; DEIMLING A ; FIGARELLA-BRANGER D ; CAVENEE WK et al.** The 2016 World Health Organization Classification of Tumors of the Central Nervous System: a summary. *Acta Neuropathol.,* 2016, vol. 131, 803-20
- **CHEN R ; SMITH-COHN M ; COHEN AL ; COLMAN H.** Glioma Subclassifications and Their Clinical Significance. *Neurotherapeutics,* 2017, vol. 14, 284-97
- **CARETTI V ; BUGIANI M ; FRERET M ; SCHELLEN P ; JANSEN M ; VAN VUURDEN D et al.** Subventricular spread of diffuse intrinsic pontine glioma. *Acta Neuropathol.,* 2014, vol. 128, 605-7
- **CASTEL D ; PHILIPPE C ; CALMON R ; LE DRET L ; TRUFFAUX N ; BODDAERT N et al.** Histone H3F3A and HIST1H3B K27M mutations define two subgroups of diffuse intrinsic pontine gliomas with different prognosis and phenotypes. *Acta Neuropathol.,* 2015, vol. 130, 815-27
- **WERBROUCK C ; EVANGELISTA CCS ; LOBÓN-IGLESIAS M-J ; BARRET E ; LE TEUFF G ; MERLEVEDE J et al.** TP53 pathway alterations drive radioresistance in Diffuse Intrinsic Pontine Gliomas (DIPG). *Clin Cancer Res.,* 2019
- **KLUIVER TA ; ALIEVA M ; VAN VUURDEN DG ; WEHRENS EJ ; RIOS AC.** Invaders Exposed: Understanding and Targeting Tumor Cell Invasion in Diffuse Intrinsic Pontine Glioma. *Front Oncol.,* 2020, vol. 10, 92
- **LIANG C-C ; PARK AY ; GUAN J-L.** In vitro scratch assay: a convenient and inexpensive method for analysis of cell migration in vitro. *Nat Protoc.,* 2007, vol. 2, 329-33

- **GUY J-B ; ESPENEL S ; VALLARD A ; BATTISTON-MONTAGNE P ; WOZNY A-S ; ARDAIL D et al.** Evaluation of the Cell Invasion and Migration Process: A Comparison of the Video Microscope-based Scratch Wound Assay and the Boyden Chamber Assay. *J Vis Exp [Internet,* 2017, https://www.ncbi.nlm.nih.gov/pmc/articles/PMC5755419
- **PLESSIER A ; LE DRET L ; VARLET P ; BECCARIA K ; LACOMBE J ; MÉRIAUX S et al.** New in vivo avatars of diffuse intrinsic pontine gliomas (DIPG) from stereotactic biopsies performed at diagnosis. *Oncotarget,* 2017, vol. 8, 52543-59
- **REYNOLDS BA ; WEISS S.** Generation of neurons and astrocytes from isolated cells of the adult mammalian central nervous system. *Science. American Association for the Advancement of Science,* 1992, vol. 255, 1707-10
- **VINCI M ; MOLINARI V ; TAYLOR K ; CARVALHO D ; BURFORD A ; MACKAY A et al.** HG-99A PATIENT-DERIVED PAEDIATRIC HIGH GRADE GLIOMA AND DIPG CELL CULTURE PANEL RECAPITULATING THE GENOTYPIC AND PHENOTYPIC DIVERSITY OF THE DISEASE. *Neuro Oncol.,* 2016, vol. 18, iii71
- **VINCI M ; BURFORD A ; MOLINARI V ; KESSLER K ; POPOV S ; CLARKE M et al.** Functional diversity and cooperativity between subclonal populations of pediatric glioblastoma and diffuse intrinsic pontine glioma cells. *Nat Med.,* 2018, vol. 24, 1204-15
- **POLLARD SM.** In vitro expansion of fetal neural progenitors as adherent cell lines. *Methods Mol Biol.,* 2013, vol. 1059, 13-24
- **POLLARD SM ; YOSHIKAWA K ; CLARKE ID ; DANOVI D ; STRICKER S ; RUSSELL R et al.** Glioma Stem Cell Lines Expanded in Adherent Culture Have Tumor-Specific Phenotypes and Are Suitable for Chemical and Genetic Screens. *Cell Stem Cell,* 2009, vol. 4, 568-80
- **HAJIAN-TILAKI K.** Receiver Operating Characteristic (ROC) Curve Analysis for Medical Diagnostic Test Evaluation. *Caspian J Intern Med.,* 2013, vol. 4, 627-35